# EUROPEAN PATENT APPLICATION

(11) **EP 3 412 202 A1**
(43) Date of publication of application: **12.12.2018**
(21) Application number: 17747314.7
(22) Date of filing: 26.01.2017
(51) Int. Cl.: A61B 5/0245, A61B 5/00, A61B 5/022

(54) **BIOLOGICAL INFORMATION EVALUATION DEVICE, BIOLOGICAL INFORMATION MEASUREMENT DEVICE, BIOLOGICAL INFORMATION EVALUATION METHOD, AND BIOLOGICAL INFORMATION EVALUATION PROGRAM**

(30) Priority: 05.02.2016 JP 2016020719
(71) Applicant: Omron Healthcare Co., Ltd., Kyoto 617-0002 (JP); Omron Corporation, Kyoto-shi, Kyoto 600-8530 (JP)
(72) Inventor: OBAYASHI Keiichi, Kyoto-shi Kyoto 600-8530 (JP); WADA Hirotaka, Kyoto-shi Kyoto 600-8530 (JP); KASAI Masaaki, Kyoto-shi Kyoto 600-8530 (JP); KOKUBO Ayako, Kyoto-shi Kyoto 600-8530 (JP); OTA Yuya, Kyoto-shi Kyoto 600-8530 (JP)
(74) Representative: Isarpatent
(86) International application number: PCT/JP2017/002791
(87) International publication number: WO 2017/135156

(57) **Abstract**

A biological information evaluation device, biological information measurement device, biological information evaluation method, and biological information evaluation program which can appropriately evaluate biological information that is varied by a circadian variation and various causes other than the circadian variation are provided. A biological information measurement device 1 which evaluates biological information that is measured from a person to be measured, and that is stored in a first storage section 13 includes: a biological information evaluating section 11D which evaluates the biological information stored in the first storage section 13, based on reference data indicating a reference value of biological information in each of split time periods which are obtained by splitting one day into a plurality of time periods; and a reference data controller 11C which controls reference data that are to be used in evaluation of the biological information by the biological information evaluating section 11D, based on at least calendar information at a measurement time of the biological information stored in the first storage section 13.

## Description

### Technical Field

The present invention relates to a biological information evaluation device, a biological information measurement device, a biological information evaluation method, and a biological information evaluation program.

### Background Art

As a method of evaluating biological information such as blood pressure information, pulse information, or beat information, for example, a method in which a preset threshold and measured biological information are compared with each other, and, if the biological information exceeds the threshold, it is determined that an abnormality occurs is usually employed.

Here, biological information such as blood pressure information, pulse information, or beat information has a large circadian variation. In the case where blood pressure information is exemplified, even when the reference blood pressure defined by Japanese Society of Hypertension or the like is set as the threshold, therefore, there is a possibility that, depending on the situation where blood pressure information is measured, the reference blood pressure does not function as an appropriate threshold. As a result, an error may possibly occur in the determination of an abnormality of the blood pressure information.

Patent Literature 1 discloses a system in which, in consideration of the circadian variation of blood pressure information, a threshold for determining a blood pressure abnormality can be finely set in unit of hour per day. In the system, the threshold in unit of hour can be manually set.

Patent Literature 2 discloses a system in which a mean value of past blood pressure information measured from a person to be measured is set as a threshold, and an abnormality of blood pressure information is determined by comparison of the threshold with measured blood pressure information.

Patent Literature 3 discloses that, in a biological information measurement device which is used in a surgery room or the like, and which measures biological information every pulse, a threshold is set based on the moving average of past measurement data measured from the person to be measured, and an abnormality of biological information is determined.

Patent Literature 4 discloses a system in which, based on results of past health examinations of a person to be measured, a reference level of biological information of the person to be measured is set.

### Citation List

### Patent Literature

Patent Literature 1: JP-A-2007-117434
Patent Literature 2: JP-A-2007-24543
Patent Literature 3: JP-A-8-256999
Patent Literature 4: JP-A-2013-248329

### Summary of Invention

### Technical Problem

In the systems disclosed in Patent Literatures 2 and 4, the circadian variation of biological information is not considered, and therefore an abnormality of biological information cannot be accurately determined.

In the biological information measurement device disclosed in Patent Literature 3, the threshold is set based on the moving average. Therefore, the device is effective in a short time period such as the surgical period, but hardly applied to a case such as that the biological information is measured for a long time period such as one day or one week.

In the system disclosed in Patent Literature 1, the threshold can be finely set in unit of hour per day, and therefore it is expected to improve the accuracy of the abnormality determination. However, the threshold is manually set, and therefore there is a possibility that the accuracy of the abnormality determination is lowered by a setting error or the like. Moreover, the work of setting the threshold is cumbersome.

It is known that biological information is varied depending on, other than the circadian variation, the atmospheric temperature, the day of week, or the like. For example, it is known that blood pressure information in winter is higher than that in summer. It is further known that blood pressure information on Monday is higher than that on the other day of week. In a case such as that where biological information is to be measured for a long time period such as one day or one week, therefore, a reference value such as a threshold for evaluating biological information must be set in consideration of these variations.

Patent Literatures 1 to 4 fail to consider variations of biological information depending on the atmospheric temperature, the day of week, or the like.

The invention has been conducted in view of the above circumstances. It is an object of the invention to provide a biological information evaluation device, biological information measurement device, biological information evaluation method, and biological information evaluation program which can appropriately evaluate biological information that is varied by a circadian variation and various causes other than the circadian variation.

### Solution to Problems

The biological information evaluation device of the present invention is a biological information evaluation device which evaluates biological information measured from a person to be measured, and stored in a first storage section, the biological information evaluation device comprising: a biological information evaluating section which evaluates the biological information stored in the first storage section, based on reference data indicating a reference value of biological information in each of split time periods which are obtained by splitting one day into a plurality of time periods; and a reference data controller which controls the reference data to be used in evaluation of the biological information by the biological information evaluating section, based on at least calendar information at a measurement time of the biological information stored in the first storage section.

The biological information measurement device of the present invention comprises: the biological information evaluation device; and a biological information measuring section which measures biological information from the person to be measured, and which stores the biological information in the first storage section.

The biological information evaluation method of the present invention is a biological information evaluation method for evaluating biological information measured from a person to be measured, and stored in a first storage section, the biological information evaluation method comprising: a biological information evaluating step of evaluating the biological information stored in the first storage section, based on reference data indicating a reference value of biological information in each of split time periods which are obtained by splitting one day into a plurality of time periods; and a reference data controlling step of controlling the reference data to be used in evaluation of the biological information in the biological information evaluating step, based on at least calendar information at a measurement time of the biological information stored in the first storage section.

The biological information evaluation program of the present invention is a biological information evaluation program for evaluating biological information measured from a person to be measured, and stored in a first storage section, the biological information evaluation program which causes a computer to execute: a biological information evaluating step of evaluating the biological information stored in the first storage section, based on reference data indicating a reference value of biological information in each of split time periods which are obtained by splitting one day into a plurality of time periods; and a reference data controlling step of controlling the reference data to be used in evaluation of the biological information in the biological information evaluating step, based on at least calendar information at a measurement time of the biological information stored in the first storage section.

### Advantageous Effects of Invention

According to the invention, it is possible to provide a biological information evaluation device, biological information measurement device, biological information evaluation method, and biological information evaluation program which can appropriately evaluate biological information that is varied by a circadian variation and various causes other than the circadian variation.

### Brief Description of Drawings

[Fig. 1] Fig. 1 is a diagram schematically showing the configuration of a biological information evaluation support system 100 for illustrating an embodiment of the invention.
[Fig. 2] Fig. 2 is a diagram showing the internal hardware configuration of a biological information measurement device 1 of the biological information evaluation support system 100 shown in Fig. 1.
[Fig. 3] Fig. 3 is a functional block diagram of a system controller 11 of the biological information measurement device 1 shown in Fig. 2.
[Fig. 4] Fig. 4 is a view showing an example of reference data.
[Fig. 5] Fig. 5 is a diagram showing the internal hardware configuration of a biological information management device 2 of the biological information evaluation support system 100 shown in Fig. 1.
[Fig. 6] Fig. 6 is a functional block diagram of a system controller 21 of the biological information management device 2 shown in Fig. 5.
[Fig. 7] Fig. 7 is a view showing an example of the data structure of a second storage section 23 of the biological information management device 2 shown in Fig. 5.
[Fig. 8] Fig. 8 is a view showing another example of the data structure of the second storage section 23 of the biological information management device 2 shown in Fig. 5.
[Fig. 9] Fig. 9 is a view showing an example of reference data to be stored in the second storage section 23 of the biological information management device 2 shown in Fig. 5.
[Fig. 10] Fig. 10 is a flowchart illustrating the operation of the biological information measurement device 1 of the biological information evaluation support system 100 shown in Fig. 1.
[Fig. 11] Fig. 11 is a diagram schematically showing the configuration of a biological information evaluation support system 100A which is a modification of the biological information evaluation support system 100 shown in Fig. 1.
[Fig. 12] Fig. 12 is a diagram showing the internal hardware configuration of a hospital terminal 4 of the biological information evaluation support system 100A shown in Fig. 11.
[Fig. 13] Fig. 13 is a functional block diagram of a system controller 41 of the hospital terminal 4 shown in Fig. 12.
[Fig. 14] Fig. 14 is a flowchart illustrating the operation of the hospital terminal 4 of the biological information evaluation support system 100A shown in Fig. 11.
[Fig. 15] Fig. 15 is a view showing a modification of reference data to be stored in the second storage section 23 of the biological information management device 2 shown in Fig. 5.
[Fig. 16] Fig. 16 is a flowchart illustrating the operation of the biological information measurement device 1 of the biological information evaluation support system 100 of the modification.
[Fig. 17] Fig. 17 is a flowchart illustrating the operation of the hospital terminal 4 of the biological information evaluation support system 100A of the modification.
[Fig. 18] Fig. 18 is a diagram showing the internal hardware configuration of a biological information measurement device 1B for illustrating an embodiment of the invention.
[Fig. 19] Fig. 19 is a functional block diagram of a system controller 11 of the biological information measurement device 1B shown in Fig. 18.
[Fig. 20] Fig. 20 is a view showing an example of a process of correcting reference data by a reference data controller 110C of the system controller 11 shown in Fig. 18.
[Fig. 21] Fig. 21 is a flowchart illustrating the operation of the biological information measurement device 1B shown in Fig. 18.
[Fig. 22] Fig. 22 is a diagram schematically showing the configuration of a biological information evaluation support system 100B for illustrating an embodiment of the invention.
[Fig. 23] Fig. 23 is a functional block diagram of a system controller 41 of a hospital terminal 4A shown in Fig. 22.
[Fig. 24] Fig. 24 is a flowchart illustrating the operation of the hospital terminal 4A of the biological information evaluation support system 100B shown in Fig. 22. Description of Embodiments

Hereinafter, embodiments of the invention will be described with reference to the drawings.

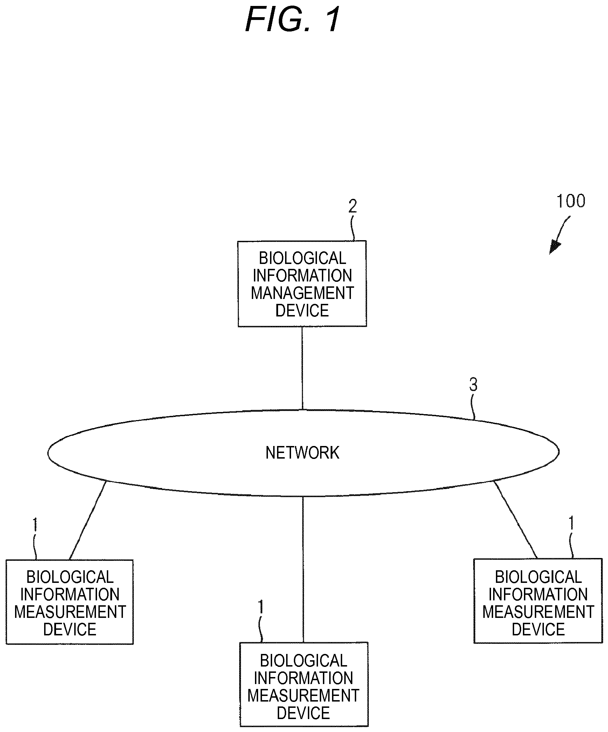

Fig. 1 is a diagram schematically showing the configuration of a biological information evaluation support system 100 for illustrating an embodiment of the invention.

The biological information evaluation support system 100 includes a plurality (in the example of Fig. 1, three) of biological information measurement devices 1, a biological information management device 2, and a network 3 such as the Internet.

Each of the plurality of biological information measurement devices 1 is configured so as to be connectable to the network 3. The biological information management device 2 is a device which is administered by an operator of the biological information evaluation support system 100, and configured so as to be connectable to the network 3.

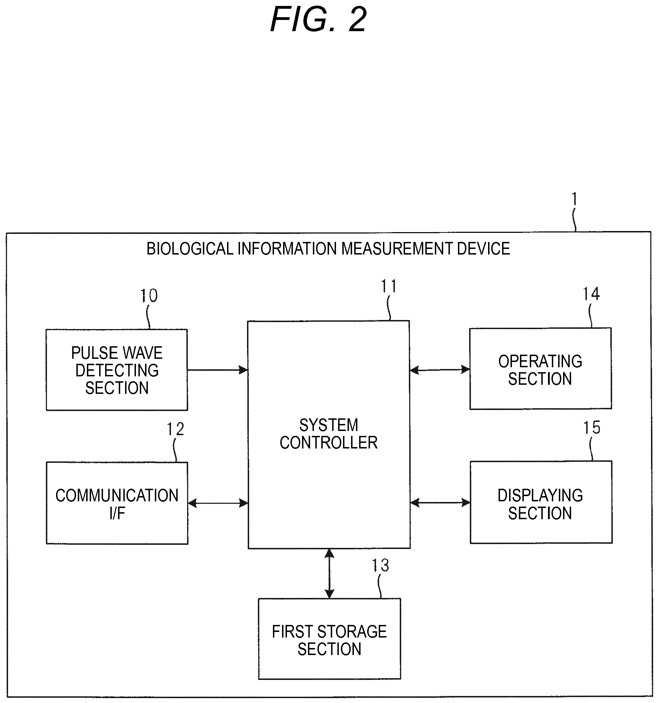

Fig. 2 is a diagram showing the internal hardware configuration of the biological information measurement device 1 of the biological information evaluation support system 100 shown in Fig. 1.

The biological information measurement device 1 measures biological information such as blood pressure information, pulse information, or beat information. For example, the blood pressure information contains the systolic blood pressure, the diastolic blood pressure, or the mean blood pressure. For example, the pulse information contains the pulse rate. For example, the beat information contains the beat rate.

The biological information measurement device 1 includes a pulse wave detecting section 10, a system controller 11 which generally controls the entire device, a communication interface (I/F) 12, a first storage section 13, an operating section 14, and a displaying section 15.

The pulse wave detecting section 10 detects a pulse wave from a living body portion (such as the wrist) of a person to be measured. In the pulse wave detecting section 10, a configuration which detects a pressure pulse wave as the pulse wave by, for example, the tonometry method is used. Alternatively, the pulse wave detecting section 10 may detect a volume pulse wave as the pulse wave. The pulse wave detecting section 10 may detect the pulse wave from reflected light from the artery which is obtained by irradiating the artery with a light beam.

The pulse wave detecting section 10 detects a pulse wave which is generated every pulse (the time period when the heart pulsates one time), and supplies the detected pulse wave to the system controller 11.

The system controller 11 is configured mainly by a processor, and includes a ROM (Read Only Memory) which stores programs and the like to be executed by the processor, a RAM (Random Access Memory) functioning as a work memory, and the like. The system controller 11 constitutes the biological information evaluation device.

The communication I/F 12 is an interface for wiredly or wirelessly connecting the device to the network 3.

The first storage section 13 is a storage medium which stores various data such as biological information measured by the biological information measurement device 1. The first storage section 13 is configured by, for example, a flash memory. The first storage section 13 may be a portable storage medium which is detachable from the biological information measurement device 1.

The operating section 14 is an interface for inputting an instruction signal for the system controller 11, and configured by buttons or touch panel for instructing start, end, and the like of various operations including measurement of biological information.

The displaying section 15 is used for displaying various kinds of information such as measured biological information, and configured by a liquid crystal display device or the like.

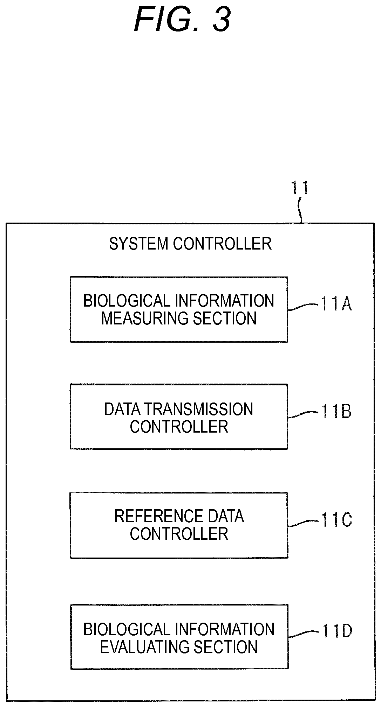

Fig. 3 is a functional block diagram of the system controller 11 of the biological information measurement device 1 shown in Fig. 2.

The system controller 11 includes a biological information measuring section 11A, a data transmission controller 11B, a reference data controller 11C, and a biological information evaluating section 11D.

The biological information measuring section 11A, the data transmission controller 11B, the reference data controller 11C, and the biological information evaluating section 11D are configured by causing the processor to execute the programs stored in the ROM. The programs include the biological information evaluation program.

The biological information measuring section 11A measures biological information by a well known method based on the pulse wave supplied from the pulse wave detecting section 10. The biological information measuring section 11A causes the measured biological information, the detection time information (hereinafter, referred to as measurement time information of biological information) of the detection of the pulse wave which is used in the measurement of the biological information, and date information and day of week information at the measurement time of the biological information, to be stored in the first storage section 13 while associating with one another.

Specifically, the biological information measuring section 11A produces measurement data including the biological information, the measurement time information, the date information, and the day of week information, and causes the measurement data to be stored in the first storage section 13.

The date information is information in which the Christian year, the month such as January to December, and date such as 1 to 31 are combined with each other, that in which the month and the date are combined with each other, information of the month, or the like.

The day of week information is information indicating the day of week such as Monday, Tuesday, ......, or Sunday. Each of the date information, and the day of week information is one of the calendar information.

The data transmission controller 11B adds the ID of the person to be measured to the measurement data (the biological information + the measurement time information + the date information + the day of week information) stored in the first storage section 13, at a predetermined timing such as that when instructions for starting measurement of biological information is given, or that when the biological information measurement device 1 is connected to the network 3, and causes the measurement data to which the ID is added, to be transmitted from the communication I/F 12 to the biological information management device 2.

The ID of the person to be measured can be manually stored in the first storage section 13 by operating the operating section 14. As the ID, for example, an ID that is allocated in a hospital to which the person to be measured visits is used.

The biological information evaluating section 11D performs evaluation of biological information contained in the measurement data which are stored in the first storage section 13, based on the reference data. The evaluation of biological information means determination whether the biological information has a value at which the health condition is paid attention or not, and specifically determination whether the biological information exceeds a reference value constituting the reference data, or not.

If the biological information exceeds the reference value, the biological information evaluating section 11D adds information (for example, a flag) indicating that the information exceeds the reference value, to the measurement data, and causes an evaluation result to be stored.

The reference data are data indicating the reference value of the biological information in each of split time periods which are obtained by splitting one day into a plurality of time periods. As the length of the split time periods, for example, an arbitrary value such as 30 minutes, one hour, or two hours is set. The split time periods may not have a same length, and the lengths of the split time periods may be dispersed.

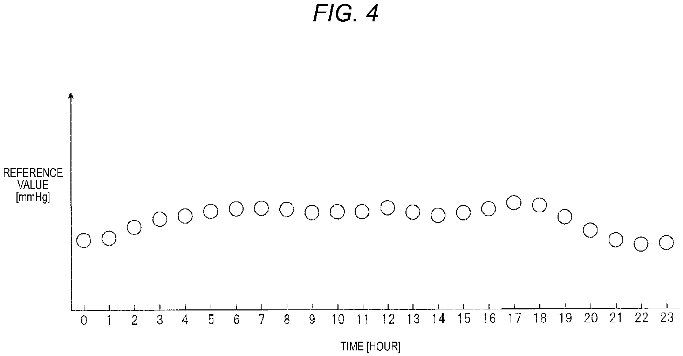

Fig. 4 is a view showing an example of the reference data.

The reference data shown in Fig. 4 are data in which one reference value is associated with respective one of split time periods that are obtained by evenly splitting one day in the unit of one hour into 24 time periods. The abscissa in Fig. 4 indicates the times in one day, and the ordinate indicates the reference value of biological information. In Fig. 4, blood pressure information is exemplified as biological information, and therefore the unit of the reference value is [mmHg].

The reference data shown in Fig. 4 have a data configuration in which N is one of 0 to 23, and one reference value is associated with an N-th hour (a time period between N o'clock and N o'clock 59 minutes 59 seconds).

The reference data controller 11C controls the above-described reference data which are used in evaluation of biological information by the biological information evaluating section 11D.

When instructions for starting measurement of biological information is issued, for example, the reference data controller 11C causes the measurement data stored in the first storage section 13, and a request signal requesting transmission of the reference data, to be transmitted from the communication I/F 12 to the biological information management device 2.

The reference data controller 11C acquires the reference data which, in response to the request signal, are transmitted from the biological information management device 2 and received by the communication I/F 12. The reference data controller 11C sets the acquired reference data as reference data (hereinafter, referred to as evaluation reference data) which are used in evaluation of biological information by the biological information evaluating section 11D.

Specifically, the reference data controller 11C adds an evaluation flag to the acquired reference data, and causes the reference data to which the evaluation flag is added, to be stored in the first storage section 13. As a result of the series of processes, the reference data are set as the evaluation reference data. The biological information evaluating section 11D evaluates biological information based on the evaluation reference data to which the evaluation flag is added.

In the case where reference data to which the evaluation flag is added exist in the first storage section 13, the reference data controller 11C causes reference data to which the evaluation flag is newly added, to be stored in the form of overwriting the reference data.

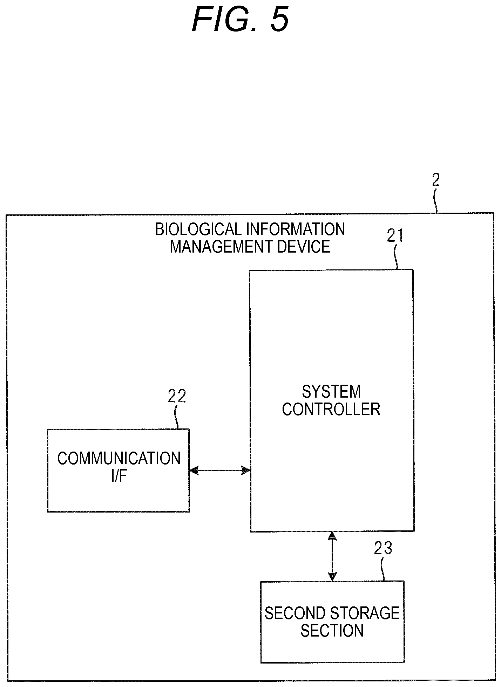

Fig. 5 is a diagram showing the internal hardware configuration of the biological information management device 2 of the biological information evaluation support system 100 shown in Fig. 1.

The biological information management device 2 includes a system controller 21 which generally controls the entire device, a communication interface (I/F) 22, and a second storage section 23.

The system controller 21 is configured mainly by a processor, and includes a ROM which stores programs and the like to be executed by the processor, a RAM functioning as a work memory, and the like.

The communication I/F 22 is an interface for wiredly or wirelessly connecting the device to the network 3.

The second storage section 23 stores the measurement data (the biological information + the measurement time information + the date information + the day of week information) and ID which are transmitted from the biological information measurement device 1 and received by the communication I/F 22, and attribute information of the person to be measured.

The second storage section 23 is configured by a storage medium such as a flash memory. The second storage section 23 is requested to exist in a place which can be accessed from the biological information management device 2, and may be configured by, for example, a storage server connected to the network 3.

The attribute information is information indicating the sex, the age, and the disease experience. For example, information indicating the disease experience includes information indicating experience of disease, and, in the case of experience, information indicating the name of the disease such as hypertension or diabetes.

The attribute information of the person to be measured, and the ID which is allocated to the person to be measured are previously stored in the second storage section 23 by a well known method. When a hospital terminal such as a personal computer which is disposed in a hospital, and which can access the biological information management device 2 is operated by a person concerned to the hospital or the person to be measured, the hospital terminal can cause the attribute information and the ID to be stored in the second storage section 23.

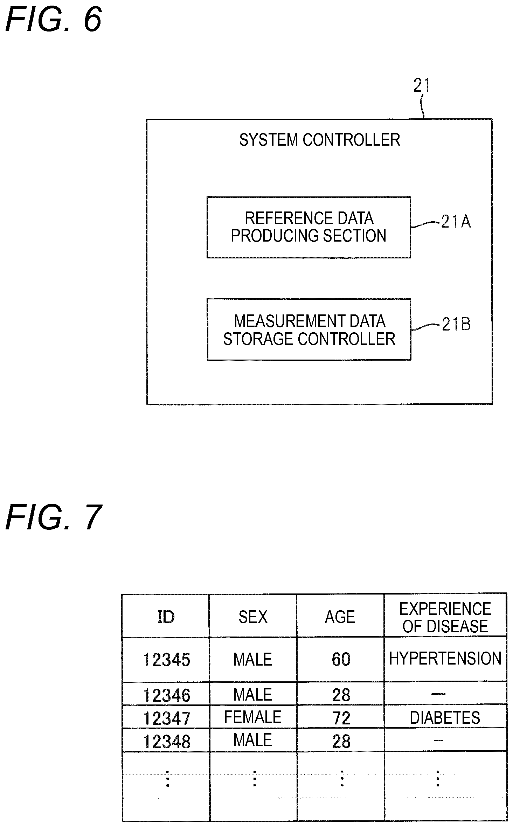

Fig. 6 is a functional block diagram of the system controller 21 of the biological information management device 2 shown in Fig. 5.

The system controller 21 includes a reference data producing section 21A, and a measurement data storage controller 21B. The reference data producing section 21A and the measurement data storage controller 21B are configured by execution of the programs stored in the ROM by the processor of the system controller 21.

The measurement data storage controller 21B performs a control in which the measurement data that are transmitted from the biological information measurement device 1 and received by the communication I/F 22 are stored in the second storage section 23.

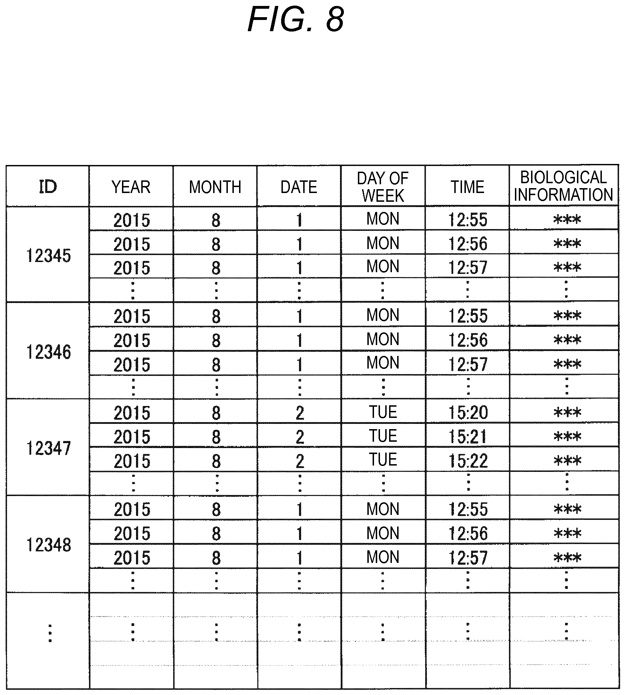

Figs. 7 and 8 are views showing examples of the data structure of the second storage section 23 of the biological information management device 2 shown in Fig. 5.

As shown in Fig. 7, the second storage section 23 stores an attribute table in which attribute information is associated with respective IDs of persons to be measured. As shown in Fig. 8, the second storage section 23 stores a measurement table in which sets of the date information, the day of week information, the measurement time information, and biological information are associated with the IDs of the persons to be measured, respectively.

The reference data producing section 21A produces reference data for each combination of the calendar information and the attribute information, based on the data of the attribute and measurement tables stored in the second storage section 23, and causes the produced reference data to be stored in the second storage section 23.

Specifically, the reference data producing section 21A refers the attribute table to select an arbitrary ID, and an ID with which attribute information that is identical with attribute information associated with the arbitrary ID is associated. In the example of Fig. 7, in the case where ID = 12346 is the arbitrary ID, ID = 12348 in which the attribute information is identical with the attribute information of ID = 12346 is selected.

Next, the reference data producing section 21A refers the measurement table to extract sets of the biological information, measurement time information, date information, and day of week information which are associated with the selected ID.

The reference data producing section 21A groups the extracted sets by the same combination of the month information and the day of week information. The reference data producing section 21A groups the biological information belonging to set groups which are obtained by the above grouping, by the same "o'clock" information (1 to 12) of the measurement time information.

The reference data producing section 21A produces the typical value of the biological information belonging to each of the time period groups which are obtained by the grouping by the "o'clock" information, as the reference value with respect to the time period group.

The typical value is a value indicating the tendency of biological information belonging to a time period group, such as the mean value of all biological information belonging to the time period group, the mode of all biological information belonging to the time period group, or the center value of all biological information belonging to the time period group.

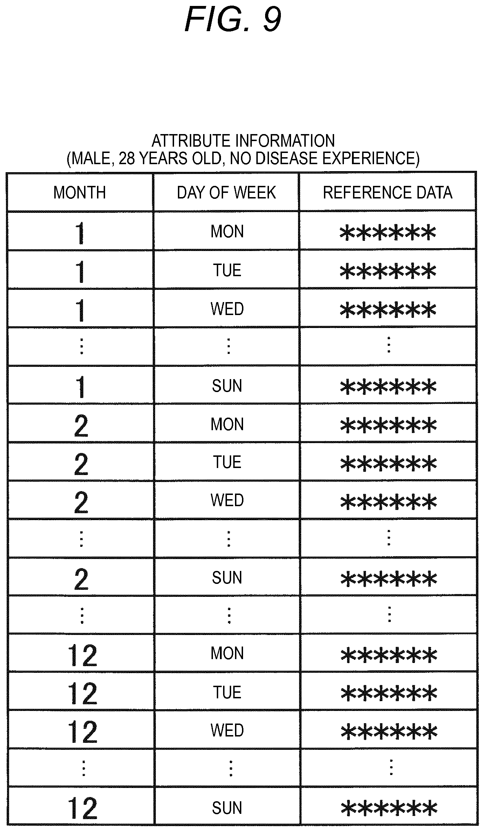

As a result of the process, the reference data such as shown in Fig. 4 are produced for each set group. For arbitrary attribute information, namely, reference data for each calendar information (combination of the month and the day of week) are stored as shown in Fig. 9. The reference data producing section 21A repeats a similar process for unselected IDs contained in the attribute table, thereby producing a reference data group shown in Fig. 9 for each of the attribute information contained in the attribute table. The reference data groups are stored in the second storage section 23.

With respect to the reference data stored in the second storage section 23, the larger number of measurement data or IDs stored in the second storage section 23, the further leveled data are produced, and the more effective as a reference for evaluation of biological information.

Next, the operation of the biological information evaluation support system 100 shown in Fig. 1 will be described.

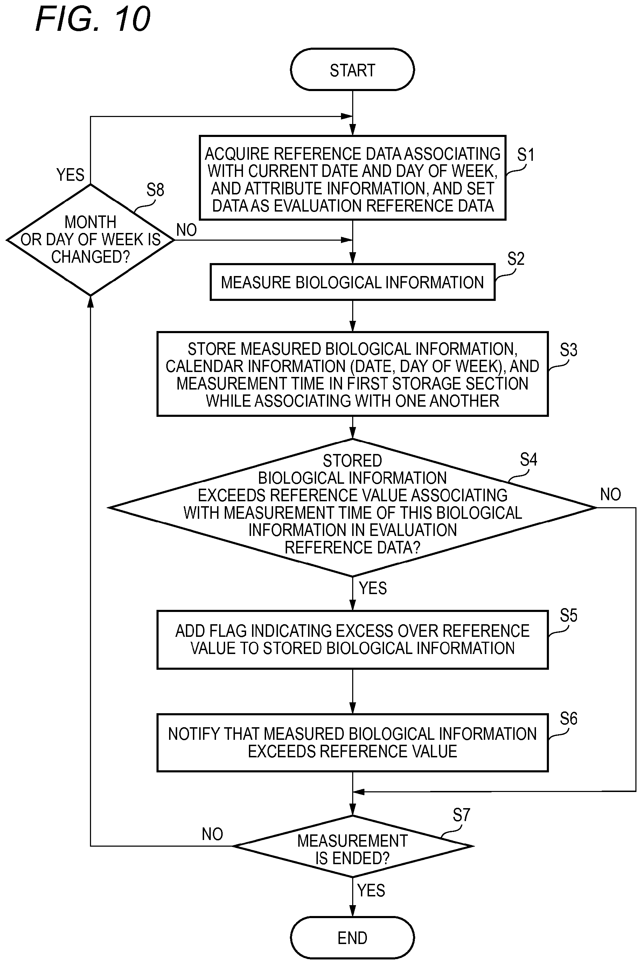

Fig. 10 is a flowchart illustrating the operation of the biological information measurement device 1 of the biological information evaluation support system 100 shown in Fig. 1.

Here, it is assumed that the communication I/F 12 of the biological information measurement device 1 is connectable to, for example, a mobile communication network, and can be connected at an arbitrary timing to the network 3 through the mobile communication network.

When the biological information measurement device 1 is attached to the person to be measured, and the person to be measured gives instructions for starting the measurement of biological information, the reference data controller 11C of the biological information measurement device 1 causes the current month information and day of week information, the ID of the person to be measured, and the request signal requesting transmission of the reference data to be transmitted to the biological information management device 2 through the communication I/F 12.

The system controller 21 of the biological information management device 2 which receives the request signal determines the attribute information of the user (person to be measured) of the biological information measurement device 1 which is the transmission source of the request signal, based on the ID of the person to be measured that is received together with the request signal, and the attribute table in the second storage section 23.

Then, the system controller 21 of the biological information management device 2 reads reference data associating with the combination of the month information and day of week information which are received together with the above-described request signal, from reference data that are stored in the second storage section 23 while associated with the determined attribute information. Thereafter, the system controller 21 of the biological information management device 2 transmits the read reference data to the biological information measurement device 1 which is the transmission source of the request signal.

The reference data controller 11C of the biological information measurement device 1 acquires the reference data which are transmitted from the biological information management device 2, and received by the communication I/F 12, and sets the reference data as evaluation reference data (step S1).

Next, the biological information measuring section 11A measures biological information from the person to be measured (step S2), and causes measurement data containing the measured biological information, the measurement time information of the biological information, and the current date information and day of week information, to be stored in the first storage section 13 (step S3).

Next, the biological information evaluating section 11D determines whether the biological information (for example, the systolic blood pressure) contained in the measurement data stored in step S3 exceeds the reference value in the evaluation reference data and associating with the measurement time information contained in the measurement data or not (step S4).

If the determination in step S4 is NO, the process is transferred to step S7.

If the determination in step S4 is YES, the biological information evaluating section 11D adds a flag indicating the excess over the reference value to the measurement data stored in step S3 (step S5).

Then, the system controller 11 of the biological information measurement device 1 notifies the person to be measured of information indicating that the measured biological information exceeds the reference value, by, for example, causing the information to be displayed on the displaying section 15 (step S6), and performs a process of step S7.

In step S7, the system controller 11 of the biological information measurement device 1 determines whether instructions for ending the measurement of biological information is given or not. If instructions for ending the measurement is given (step S7: YES), the measurement operation is ended.

If instructions for ending the measurement is not given (step S7: NO), the system controller 11 of the biological information measurement device 1 checks the current month information and day of week information, and, after the process of step S1 is performed, determines whether the month information or the day of week information is changed or not (step S8).

Usually, the timing when the date or the day of week is changed is 0 o'clock AM. Alternatively, the timing may be set to a predetermined time such as 6 o'clock AM, and, if it is the time after the process of step S1 is performed, the system controller 11 may determine that the date or the day of week is changed.

If the determination in step S8 is YES, the process is returned to step S1, and, if the determination in step S8 is NO, the process is returned to step S2.

As described above, according to the biological information evaluation support system 100, the evaluation reference data which are to be used for evaluating biological information are data having reference values for respective split time periods which are obtained by splitting one day into a plurality of time periods, and therefore biological information can be evaluated by using the optimum reference value in which a circadian variation of biological information is considered.

Moreover, the evaluation reference data are not always same, and are adequately changed in accordance with the month or day of week information at the time of measurement of biological information. Therefore, it is possible to perform evaluation of biological information by using the optimum reference value in which variation of biological information due to the difference in the atmospheric temperature, the difference between the days of week, or the like is considered.

Moreover, biological information is largely varied depending on the difference in sex, that in age, or the presence or absence of chronic disease. However, the biological information measurement device 1 can evaluate biological information based on reference data which are produced from biological information of a parson having the same attribute information as the person to be measured. Therefore, biological information can be evaluated by using the optimum reference value in which attribute information of each person is considered.

In step S3 of the flowchart shown in Fig. 10, alternatively, the biological information measuring section 11A may cause the evaluation reference data which are set in step S1, to be stored in the first storage section 13 while associated with the measured biological information. According to the configuration, it can be later checked what kind of reference data is used in evaluation of biological information, and this is useful in diagnosis or the like.

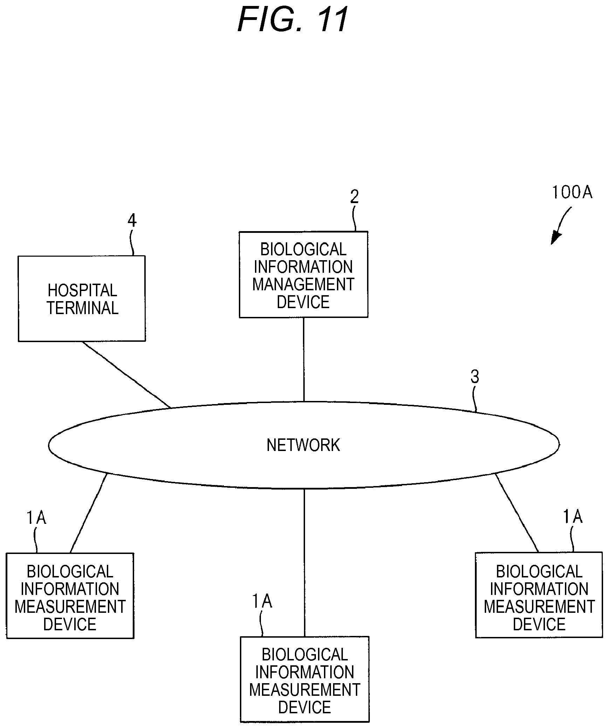

Fig. 11 is a diagram schematically showing the configuration of a biological information evaluation support system 100A which is a modification of the biological information evaluation support system 100 shown in Fig. 1.

The biological information evaluation support system 100A shown in Fig. 11 is configured in the same manner as the biological information evaluation support system 100 shown in Fig. 1 except that the biological information measurement device 1 is changed to a biological information measurement device 1A, and a hospital terminal 4 is added. Therefore, the components identical with those of Fig. 1 are denoted by the same reference numerals, and their description is omitted.

In the biological information evaluation support system 100A, it is assumed that biological information is not evaluated in the biological information measurement device 1A, but is evaluated in the hospital terminal 4.

For example, the patient who occasionally visits a hospital measures biological information by using the biological information measurement device 1A for a predetermined time period, and then brings the biological information measurement device 1A to the hospital. In the hospital, the measurement data are read from the biological information measurement device 1A into the hospital terminal 4, evaluation of the biological information and display of a result of the evaluation are performed in the hospital terminal 4, and the doctor performs diagnosis with reference to the evaluation result.

The hospital terminal 4 is an electronic device which can access the biological information management device 2 through the network 3, such as a personal computer or tablet terminal which is disposed in the hospital.

The hospital terminal 4 can be wiredly or wirelessly connected to the biological information measurement device 1, and read data of the first storage section 13. In the case where the first storage section 13 is a portable storage medium, the hospital terminal 4 may have means by which data can be read from the storage medium, and be configured so as to read data from a loaded storage medium.

The internal hardware configuration of the biological information measurement device 1A is identical with that shown in Fig. 2, and its description is omitted. In a functional block diagram of the system controller 11 of the biological information measurement device 1A, the reference data controller 11C and the biological information evaluating section 11D are deleted from Fig. 3.

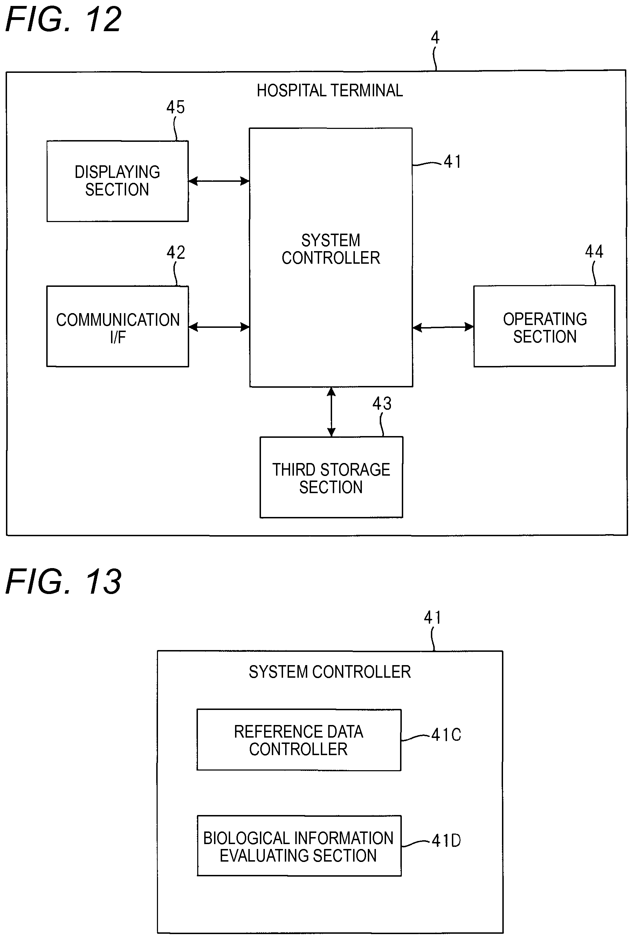

Fig. 12 is a diagram showing the internal hardware configuration of the hospital terminal 4 of the biological information evaluation support system 100A shown in Fig. 11.

The hospital terminal 4 includes a system controller 41 which generally controls the entire terminal, a communication interface (I/F) 42, a third storage section 43, an operating section 44, and a displaying section 45.

The system controller 41 is configured mainly by a processor, and includes a ROM which stores programs and the like to be executed by the processor, a RAM functioning as a work memory, and the like.

The communication I/F 42 includes an interface for wiredly or wirelessly connecting the terminal to the network 3, and that for wiredly or wirelessly connecting the terminal to an electronic device including the biological information measurement device 1A.

The third storage section 43 is a storage medium which stores data that are read from the biological information measurement device 1A. The third storage section 43 is configured by a storage medium such as a flash memory. The third storage section 43 is requested to exist in a place which can be accessed from the hospital terminal 4, and may be configured by, for example, a storage server connected to the network 3.

The operating section 44 is an interface for inputting an instruction signal for the system controller 41, and configured by a keyboard, a mouse, buttons, a touch panel, or the like.

The displaying section 45 is used for displaying various kinds of information such as biological information stored in the third storage section 43, and configured by a liquid crystal display device or the like.

Fig. 13 is a functional block diagram of the system controller 41 of the hospital terminal 4 shown in Fig. 12.

The system controller 41 includes a reference data controller 41C and a biological information evaluating section 41D.

The reference data controller 41C and the biological information evaluating section 41D are configured by causing the processor to execute the programs stored in the ROM. The programs include the biological information evaluation program. The system controller 41 constitutes the biological information evaluation device.

The reference data controller 41C sets reference data which are used in evaluation by the biological information evaluating section 41D.

The biological information evaluating section 41D evaluates biological information which is acquired from the biological information measurement device 1A and stored in the third storage section 43, based on the reference data (evaluation reference data) which are set by the reference data controller 41C.

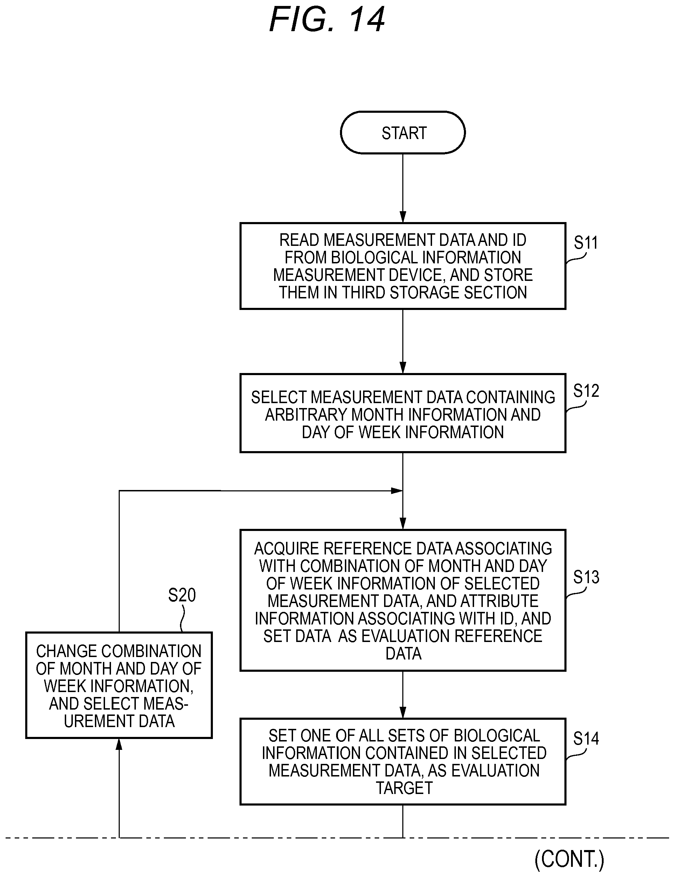

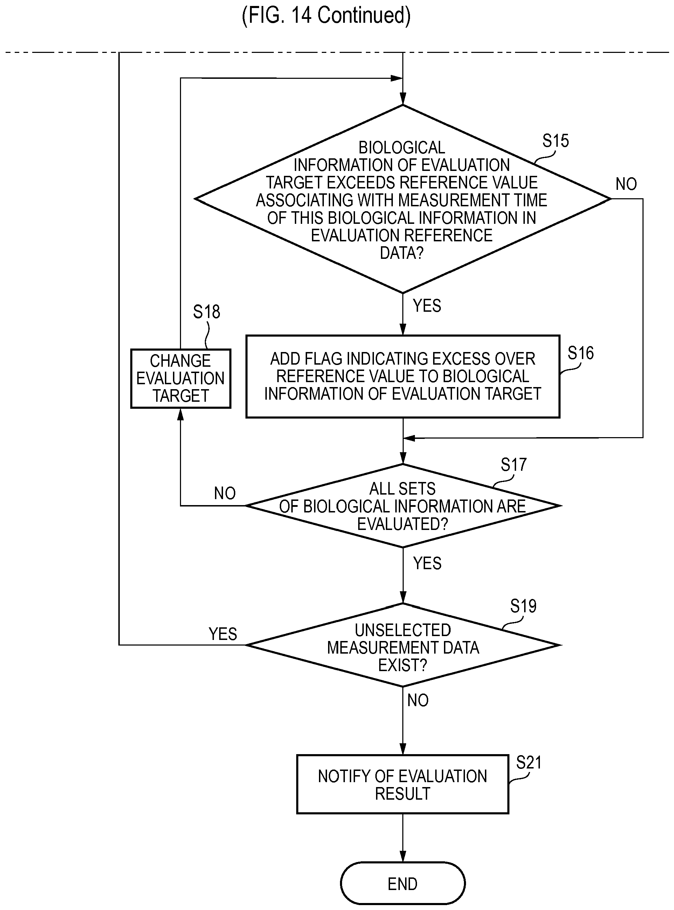

Fig. 14 is a flowchart illustrating the operation of the hospital terminal 4 of the biological information evaluation support system 100A shown in Fig. 11.

First, the biological information measurement device 1A is brought into the hospital by the person to be measured, and connected to the hospital terminal 4 which is disposed in the hospital. In this state, the system controller 41 of the hospital terminal 4 reads the measurement data and ID of the person to be measured which are stored in the first storage section 13 of the biological information measurement device 1A, and causes the read measurement data and ID to be stored in the third storage section 43 (step S11).

Next, the reference data controller 41C of the system controller 41 selects measurement data including arbitrary month information and arbitrary day of week information, from the measurement data stored in the third storage section 43 (step S12).

Next, the reference data controller 41C of the system controller 41 transmits the month information and day of week information included in the selected measurement data, the ID stored in the third storage section 43, and a request signal requesting transmission of the reference data, to the biological information management device 2.

The system controller 21 of the biological information management device 2 which receives the request signal determines the attribute information associating with the ID of the person to be measured that is received together with the request signal, based on the ID and the attribute table in the second storage section 23.

Then, the system controller 21 of the biological information management device 2 reads reference data associating with the combination of the month information and day of week information which are received together with the above-described request signal, from reference data that are stored in the second storage section 23 while associated with the determined attribute information. Thereafter, the system controller 21 of the biological information management device 2 transmits the read reference data to the hospital terminal 4 which is the transmission source of the request signal.

The reference data controller 41C of the hospital terminal 4 acquires the reference data which are transmitted from the biological information management device 2, and which are received by the communication I/F 42, and sets the reference data as evaluation reference data (step S13).

Next, the biological information evaluating section 41D of the hospital terminal 4 sets one of sets of biological information contained in the measurement data selected in step S12, as the evaluation target (step S14).

Next, the biological information evaluating section 41D of the hospital terminal 4 determines whether the biological information (for example, the systolic blood pressure) of the evaluation target exceeds the reference value in the evaluation reference data set in step S13 and associating with the measurement time information of the biological information or not (step S15).

If the determination in step S15 is NO, the process is transferred to step S17.

If the determination in step S15 is YES, the biological information evaluating section 41D of the hospital terminal 4 adds a flag indicating the excess over the reference value to the biological information of the evaluation target (step S16), and transfers the process to step S17.

In step S17, the biological information evaluating section 41D of the hospital terminal 4 determines whether all sets of biological information contained in the selected measurement data are set as an evaluation target or not.

If the determination in step S17 is NO, the biological information evaluating section 41D of the hospital terminal 4 changes biological information which is to be an evaluation target, to information which has not yet been set as an evaluation target (step S18), and returns the process to step S15.

If the determination in step S17 is YES, the biological information evaluating section 41D of the hospital terminal 4 determines whether unselected measurement data exist in the measurement data stored in the third storage section 43 or not (step S19).

If the determination in step S19 is YES, the biological information evaluating section 41D of the hospital terminal 4 changes the combination of the month information and the day of week information to another combination, selects measurement data including the month information and day of week information which have been changed (step S20), and returns the process to step S13.

If the determination in step S19 is NO, the system controller 41 of the hospital terminal 4 notifies of a result of the measurement of biological information (step S21), and ends the process.

The system controller 41 of the hospital terminal 4 causes the displaying section 45 to display, for example, a graph in which the sets of evaluated biological information are set as the ordinate, and the measurement times are set as the abscissa, and biological information which exceeds the reference value to be highlighted in the graph.

As described above, according to the biological information evaluation support system 100A, biological information can be adequately evaluated similarly with the biological information evaluation support system 100.

According to the biological information evaluation support system 100A, moreover, the function of the biological information measurement device 1A can be simplified, and the production cost and power consumption of the biological information measurement device 1A can be reduced.

In the biological information evaluation support system 100 shown in Fig. 1, it is assumed that the biological information measurement device 1 performs in parallel the measurement of biological information and the evaluation of biological information. The timing when the biological information measurement device 1 evaluates biological information may be not during the measurement of biological information.

In this case, the system controller 11 of the biological information measurement device 1 may start the processes of step S12 and subsequent steps shown in Fig. 14, at, for example, a timing when the measurement of biological information is ended or the person to be measured gives instructions, while targeting the measurement data stored in the first storage section 13.

In the case where, as in the operation example shown in Fig. 10, the biological information measurement device 1 performs in parallel the measurement of biological information and the evaluation of biological information, notification to the person to be measured can be conducted in real time at a timing when the biological information exceeds the reference value. Therefore, the system is effective for a person who must strictly manage biological information.

In the above description, it is assumed that the reference data are produced for each combination of the month information, the day of week information, and the attribute information, and stored in the second storage section 23. In a modification, the reference data may be produced for each combination of the atmospheric temperature information, the day of week information, and the attribute information, and stored in the second storage section 23. Hereinafter, the modification will be described.

In the modification, the biological information measurement device 1 or the biological information measurement device 1A has a temperature sensor which detects the ambient temperature (atmospheric temperature) of the device at a timing when biological information is measured.

In the modification, the biological information measuring section 11A of the biological information measurement device 1 or the biological information measurement device 1A causes the measured biological information, the measurement time information of the biological information, date information and day of week information at the measurement time of the biological information, and the atmospheric temperature information at the measurement time of the biological information, to be stored in the first storage section 13 while associating with one another. The biological information measuring section 11A produces the above atmospheric temperature information from information of the temperature detected by the temperature sensor.

The atmospheric temperature information may be the temperature itself detected by the temperature sensor, or information indicating one of a plurality of predetermined steps of atmospheric temperature ranges to which the temperature detected by the temperature sensor belongs.

In the case where five steps of 0°C or lower, 0°C to 10°C, 10°C to 20°C, 20°C to 30°C, and 30°C or higher are set as atmospheric temperature ranges, when the temperature detected by the temperature sensor is 26°C, for example, information indicating the temperature range of 20°C to 30°C is produced as the atmospheric temperature information.

In the modification, the reference data producing section 21A of the biological information management device 2 produces reference data for each combination of the calendar information, the attribute information, and the atmospheric temperature information, based on the data of the attribute and measurement tables stored in the second storage section 23, and causes the produced reference data to be stored in the second storage section 23.

Specifically, the reference data producing section 21A refers the attribute table to select an arbitrary ID, and an ID with which attribute information that is identical with attribute information associated with the arbitrary ID is associated. Next, the reference data producing section 21A refers the measurement table to extract sets of the biological information, measurement time information, date information, day of week information, and atmospheric temperature information which are associated with the selected ID.

The reference data producing section 21A groups the extracted sets by the same combination of the day of week information and the atmospheric temperature information. The reference data producing section 21A groups the biological information belonging to set groups which are obtained by the above grouping, by the same "o'clock" information (1 to 12) of the measurement time information. The reference data producing section 21A produces the typical value of the biological information belonging to each of the time period groups which are obtained by the grouping by the "o'clock" information, as the reference value with respect to the time period group.

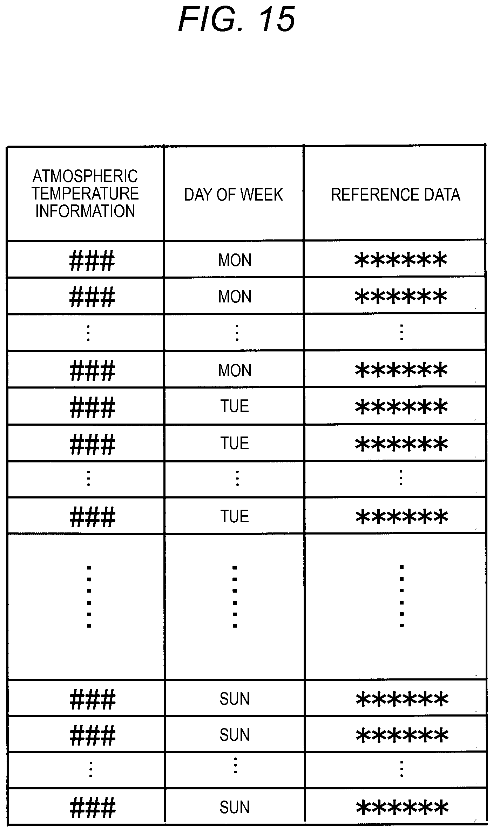

As a result of the process, with respect to arbitrary attribute information, the reference data for each combination of the day of week information and the atmospheric temperature information are stored as shown in Fig. 15. The reference data producing section 21A repeats a similar process for unselected IDs contained in the attribute table, thereby producing a reference data group shown in Fig. 15 for each of all sets of the attribute information contained in the attribute table. The reference data groups are stored in the second storage section 23.

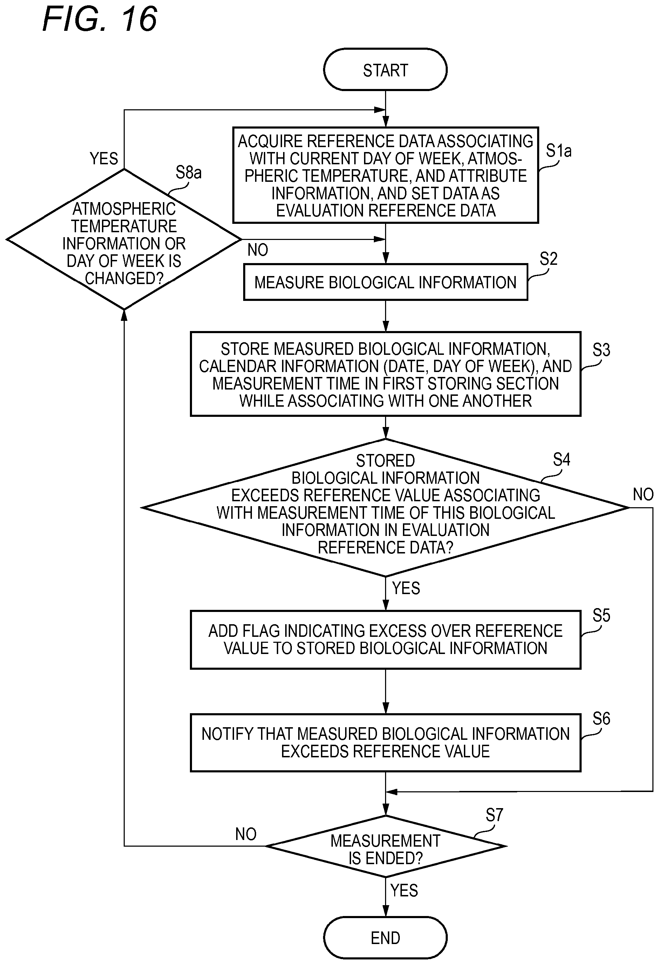

Fig. 16 is a flowchart illustrating the operation of the biological information measurement device 1 of the biological information evaluation support system 100 of the modification. In Fig. 16, the processes identical with those of Fig. 10 are denoted by the same reference numerals, and their description is omitted.

When the biological information measurement device 1 is attached to the person to be measured, and the person to be measured gives instructions for starting the measurement of biological information, the reference data controller 11C of the biological information measurement device 1 causes the current atmospheric temperature information and day of week information, the ID of the person to be measured, and the request signal requesting transmission of the reference data to be transmitted to the biological information management device 2 through the communication I/F 12.

The system controller 21 of the biological information management device 2 which receives the request signal determines the attribute information of the user (person to be measured) of the biological information measurement device 1 which is the transmission source of the request signal, based on the ID of the person to be measured that is received together with the request signal, and the attribute table in the second storage section 23.

Then, the system controller 21 of the biological information management device 2 reads reference data associating with the combination of the atmospheric temperature information and day of week information which are received together with the above-described request signal, from reference data that are stored in the second storage section 23 while associating with the determined attribute information. Thereafter, the system controller 21 of the biological information management device 2 transmits the read reference data to the biological information measurement device 1 which is the transmission source of the request signal.

The reference data controller 11C of the biological information measurement device 1 acquires the reference data which are transmitted from the biological information management device 2, and received by the communication I/F 12, and sets the reference data as evaluation reference data (step S1a). After step S1a, the processes of steps S2 to S7 are performed. If the determination in step S7 is NO, the system controller 11 of the biological information measurement device 1 checks the current atmospheric temperature information and day of week information, and, after the process of step S1 is performed, determines whether the atmospheric temperature information or the day of week information is changed or not (step S8a).

If the determination in step S8a is YES, the system controller 11 returns the process to step S1a, and, if the determination in step S8a is NO, returns the process to step S2.

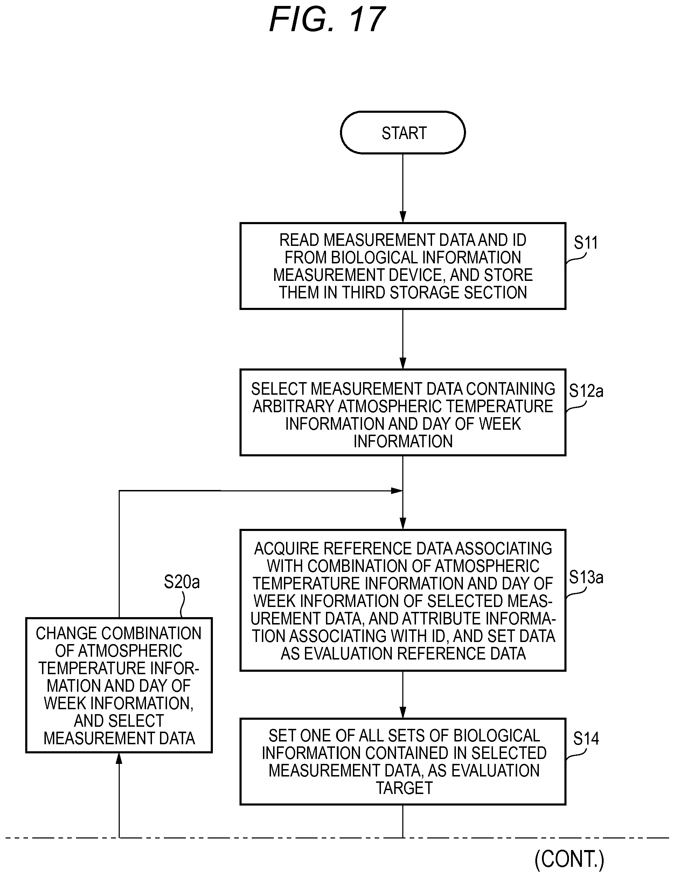

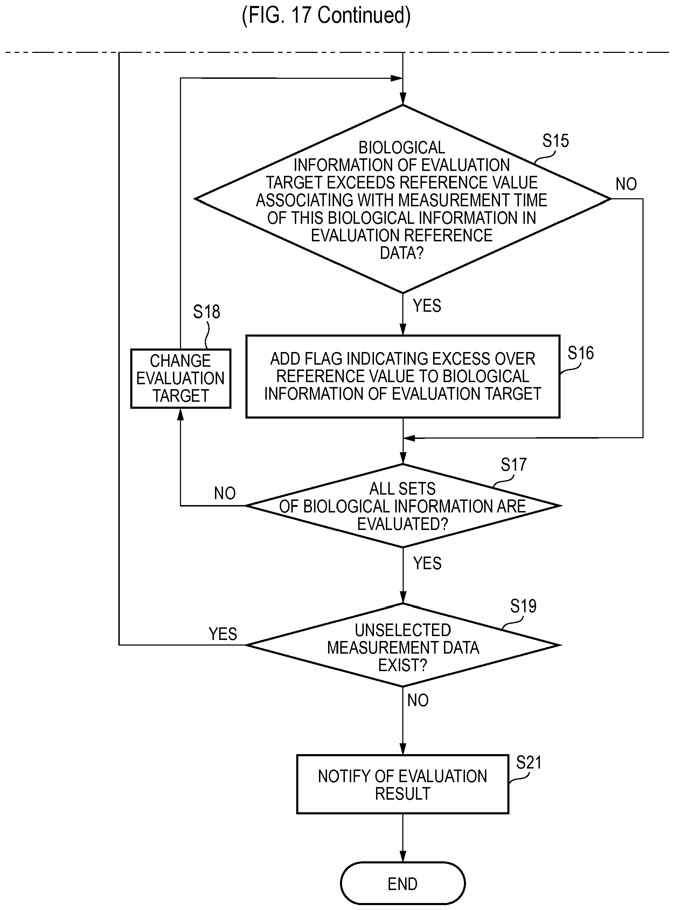

Fig. 17 is a flowchart illustrating the operation of the hospital terminal 4 of the biological information evaluation support system 100A of the modification. In Fig. 17, the processes identical with those of Fig. 14 are denoted by the same reference numerals, and their description is omitted.

After step S11, the reference data controller 41C of the system controller 41 selects measurement data containing arbitrary atmospheric temperature information and arbitrary day of week information, from the measurement data stored in the third storage section 43 (step S12a).

Next, the reference data controller 41C of the system controller 41 transmits the atmospheric temperature information and day of week information included in the selected measurement data, the ID stored in the third storage section 43, and a request signal requesting transmission of the reference data, to the biological information management device 2.

The system controller 21 of the biological information management device 2 which receives the request signal determines the attribute information associating with the ID of the person to be measured that is received together with the request signal, based on the ID and the attribute table in the second storage section 23.

Then, the system controller 21 of the biological information management device 2 reads reference data associating with the combination of the atmospheric temperature information and day of week information which are received together with the above-described request signal, from reference data that are stored in the second storage section 23 while associating with the determined attribute information. Thereafter, the system controller 21 of the biological information management device 2 transmits the read reference data to the hospital terminal 4 which is the transmission source of the request signal.

The reference data controller 41C of the hospital terminal 4 acquires the reference data which are transmitted from the biological information management device 2, and which are received by the communication I/F 42, and sets the reference data as evaluation reference data (step S13a). After step S13a, the processes of steps S14 to S19 are performed.

If the determination in step S19 is YES, the system controller 41 of the hospital terminal 4 changes the combination of the atmospheric temperature information and the day of week information to another combination, selects measurement data containing the atmospheric temperature information and day of week information which have been changed (step S20a), and returns the process to step S13a.

According to the system of the modification, as described above, reference data are previously produced and stored for each combination of the atmospheric temperature information, the day of week information, and the attribute information, and biological information can be evaluated by using reference data associating with the atmospheric temperature information at measurement of the biological information, the attribute information of the person to be measured of the biological information, and the day of week information at the measurement time of the biological information. When blood pressure information is assumed as biological information, the blood pressure information is largely changed in accordance with the atmospheric temperature. When biological information is evaluated by using reference data in which the atmospheric temperature information at the measurement time of the biological information is considered, therefore, the biological information can be adequately evaluated.

In the above description, it is assumed that the biological information management device 2 produces reference data based on measurement data of a plurality of persons which are measured by the plurality of biological information measurement devices 1 (biological information measurement devices 1A). As a modification of this, a mode in which the biological information measurement device 1 produces reference data will be described.

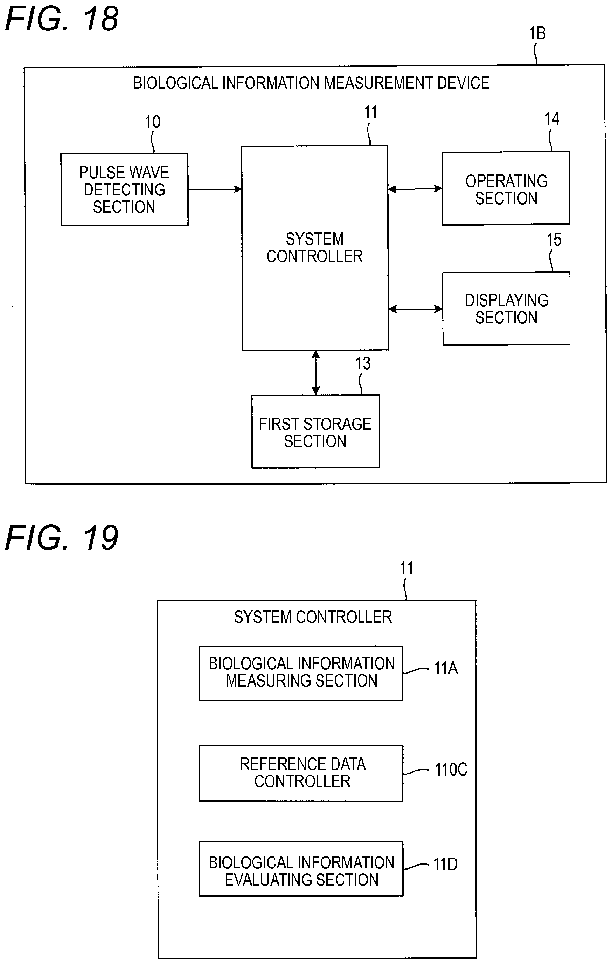

Fig. 18 is a diagram showing the internal hardware configuration of a biological information measurement device 1B for illustrating an embodiment of the invention. The hardware configuration of the biological information measurement device 1B shown in Fig. 18 is identical with that of the biological information measurement device 1 shown in Fig. 2, except that the communication I/F 12 is deleted. Therefore, the configurations identical with those of Fig. 2 are denoted by the same reference numerals, and their description is omitted.

Fig. 19 is a functional block diagram of the system controller 11 of the biological information measurement device 1B shown in Fig. 18. The system controller 11 shown in Fig. 19 has a configuration where, in Fig. 3, the data transmission controller 11B is deleted, and the reference data controller 11C is changed to a reference data controller 110C. Therefore, the configurations identical with those of Fig. 3 are denoted by the same reference numerals, and their description is omitted.

The reference data controller 110C is configured by causing the processor to execute the programs stored in the ROM. The programs include the biological information evaluation program. The system controller 11 of the biological information measurement device 1B constitutes the biological information evaluation device.

When the measurement data which are measured by the biological information measuring section 11A, and which are stored in the first storage section 13 are accumulated for seven days, the reference data controller 110C produces reference data for each day of week based on the measurement data for seven days, and causes the produced reference data, and information of the measurement month of the biological information which is used in production of the reference data, to be stored in the first storage section 13 while associating with each other. The reference data for one day mean data which are obtained by consecutively performing a measurement for a time period from 0 o'clock to 23 o'clock 59 minutes.

Specifically, the reference data controller 110C produces the typical value of the biological information belonging to each of the time period groups which are obtained by grouping the biological information contained in measurement data for one day that are measured on Mondays of the same month, by the "o'clock" information, as the reference value with respect to the time period group. As a result of the process, reference data associating with Monday are produced. The reference data controller 110C causes the reference data associating with Monday, and the month information contained in the measurement data used in production of the reference data, to be stored in the first storage section 13 while associating with each other.

The reference data controller 110C performs a similar process on measurement data which are measured on the other days of week, to produce and store reference data associating with each of Monday to Sunday.

When biological information is to be evaluated, the reference data controller 110C corrects reference data associating with the day of week information which is associating with the biological information that is the evaluation target, among reference data stored in the first storage section 13, based on the month information which is associated with the biological information that is the evaluation target, and sets the corrected reference data as evaluation reference data.

When blood pressure information is exemplified as biological information, blood pressure information in winter tends to be higher than that in summer. Such seasonal variation is not considered in the reference data for each day of week which are produced by the reference data controller 110C, and which are stored in the first storage section 13.

In an example case where the biological information measurement device 1B measures blood pressure information on arbitrary Tuesday of August, even when biological information is evaluated based on reference data associating with Tuesday, therefore, there is a possibility that the evaluation cannot be correctly performed in the case where the reference data are produced based on biological information which is measured on arbitrary Tuesday of December.

Therefore, the reference data controller 110C corrects the evaluation reference data to data according to the month of the measurement time, based on the relationship between the month information at the measurement time of the biological information, and the information of the measurement month of the biological information which is used in production of the evaluation reference data.

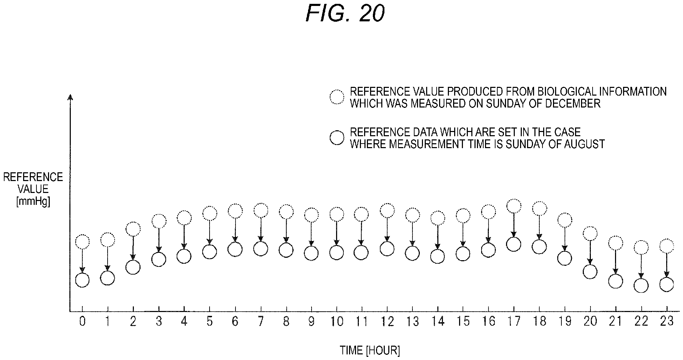

Fig. 20 is a view showing an example of a process of correcting reference data by the reference data controller 110C of the system controller 11 shown in Fig. 18.

In the example of Fig. 20, the case is shown where biological information is measured on Sunday of August, the reference data are corrected by uniformly reducing reference values of reference data which were produced based on biological information which was measured on Sunday of December.

In order to realize such correction, a table is previously stored in the first storage section 13. The table stores correction values indicating degrees of increasing or decreasing reference values of reference data, with respect to combinations of the month information contained in measurement data used in the production of the reference data, and the month information associated with the biological information which is the evaluation target.

Based on the table, the reference data controller 110C then reads the correction value associating with the combination of the month information associated with the biological information which is the evaluation target, and the month information contained in measurement data used in the production of the reference data, and corrects the reference data in accordance with the read correction value. Therefore, biological information can be evaluated in consideration of variation of biological information due to the difference in season.

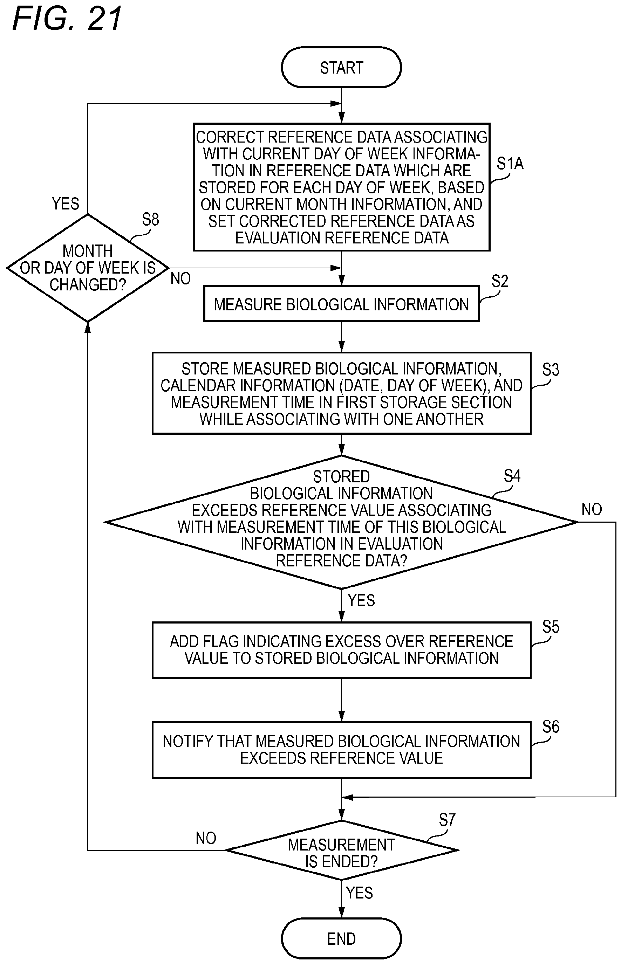

Fig. 21 is a flowchart illustrating the operation of the biological information measurement device 1B shown in Fig. 18. The flowchart shown in Fig. 21 is identical with that shown in Fig. 10 except that step S1 is changed to step S1A. Therefore, the processes identical with those of Fig. 10 are denoted by the same reference numerals, and their description is omitted. Here, it is premised that reference data associating with respective days of week from Monday to Sunday are previously stored in the first storage section 13 of the biological information measurement device 1B.

When instructions for starting measurement of biological information is given, the reference data controller 110C reads reference data associating with the current day of week information, and the month information associated with this, from the first storage section 13.

Next, the reference data controller 110C refers the table stored in the first storage section 13, and reads the correction value associating with the combination of the month information at the present time, and the above read month information. Then, the reference data controller 110C corrects the read reference data based on the read correction value, and sets the corrected reference data as the evaluation reference data (step S1A). After the process of step S1A, the processes of step S2 and subsequent steps are performed.

According to the biological information measurement device 1B, as described above, the device alone can produce reference data. Therefore, the cost for structuring a system can be eliminated, and the low cost can be realized. According to the biological information measurement device 1B, moreover, biological information is evaluated based on reference data which are produced based on the biological information of the user oneself of the biological information measurement device 1B, and therefore the health condition can be evaluated more correctly.

The function of the reference data controller 110C of the biological information measurement device 1B may be changed in the following manner.

When the measurement data which are measured by the biological information measuring section 11A, and which are stored in the first storage section 13 are accumulated for one day, namely, the reference data controller 110C produces one set of reference data based on measurement data for one day in which the month information and the day of week information are identical, and stores the produced reference data, and the month information and day of week information that are associated with the biological information used in the production of the reference data, in the first storage section 13 while associating with each other.

When biological information is to be evaluated, then, the reference data controller 110C corrects the reference data stored in the first storage section 13, based on the month information and day of week information which are associated with the biological information that is the evaluation target, and sets the corrected reference data as the evaluation reference data.

In this case, a table storing correction values indicating degrees of increasing or decreasing reference values of reference data, with respect to combinations of the month information and day of week information which are associated with the reference data, and the month information and day of week information which are associated with the biological information that is the evaluation target is previously stored in the first storage section 13.

Based on the table, the reference data controller 110C reads a correction value corresponding to the combination of the month information and day of week information which are associated with the biological information that is the evaluation target, and the month information and day of week information which associate with the reference data, and corrects the reference data in accordance with the read correction value. This enables biological information to be evaluated in consideration of variation of biological information due to the difference in season and the difference in day of week.

According to the configuration, when biological information is measured only for one day, the biological information can be correctly evaluated. Therefore, this is effective in the case where biological information is to be evaluated in a hurry.

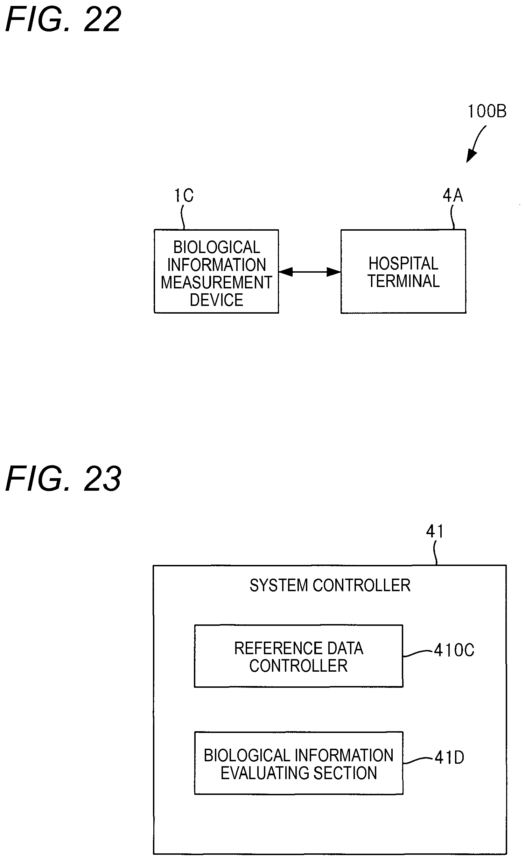

Fig. 22 is a diagram schematically showing the configuration of a biological information evaluation support system 100B for illustrating an embodiment of the invention. The biological information evaluation support system 100B includes a biological information measurement device 1C and a hospital terminal 4A.

The internal hardware configuration of the biological information measurement device 1C is identical with that of Fig. 2. In the functional block diagram of the system controller 11 of the biological information measurement device 1C, the data transmission controller 11B, the reference data controller 11C, and the biological information evaluating section 11D are deleted from that of Fig. 3.

The hardware configuration of the hospital terminal 4A is identical with that of Fig. 12.

Fig. 23 is a functional block diagram of a system controller 41 of the hospital terminal 4A shown in Fig. 22.

The system controller 41 of the hospital terminal 4A has the same configuration as that of Fig. 13 except that the reference data controller 41C is changed to a reference data controller 410C. Therefore, the configurations identical with those of Fig. 13 are denoted by the same reference numerals, and their description is omitted.

The reference data controller 410C is configured by causing the processor to execute the programs stored in the ROM. The programs include the biological information evaluation program. The system controller 41 of the hospital terminal 4A constitutes the biological information evaluation device.

The reference data controller 410C produces reference data for each day of week based on the measurement data for seven days which are read from the biological information measurement device 1C, and which are stored in the third storage section 43 of the hospital terminal 4A, and causes the produced reference data to be stored in the third storage section 43. The method of producing the reference data is identical with that described above.

The reference data controller 410C corrects the reference data stored in the third storage section 43, based on the month information of the biological information which is the evaluation target, and sets the corrected reference data as the evaluation reference data. The correction method is identical with that described before.

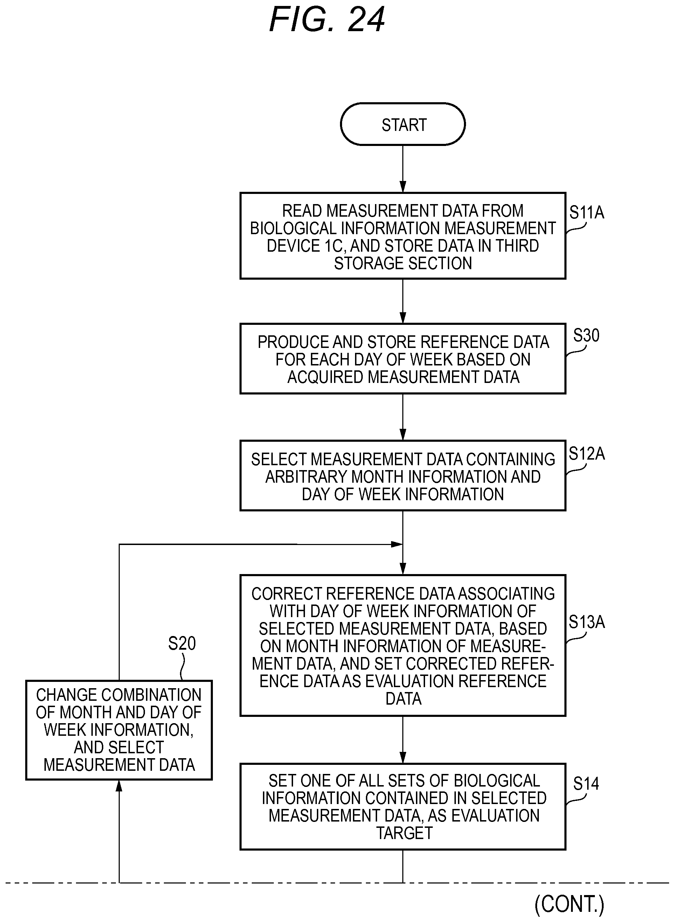

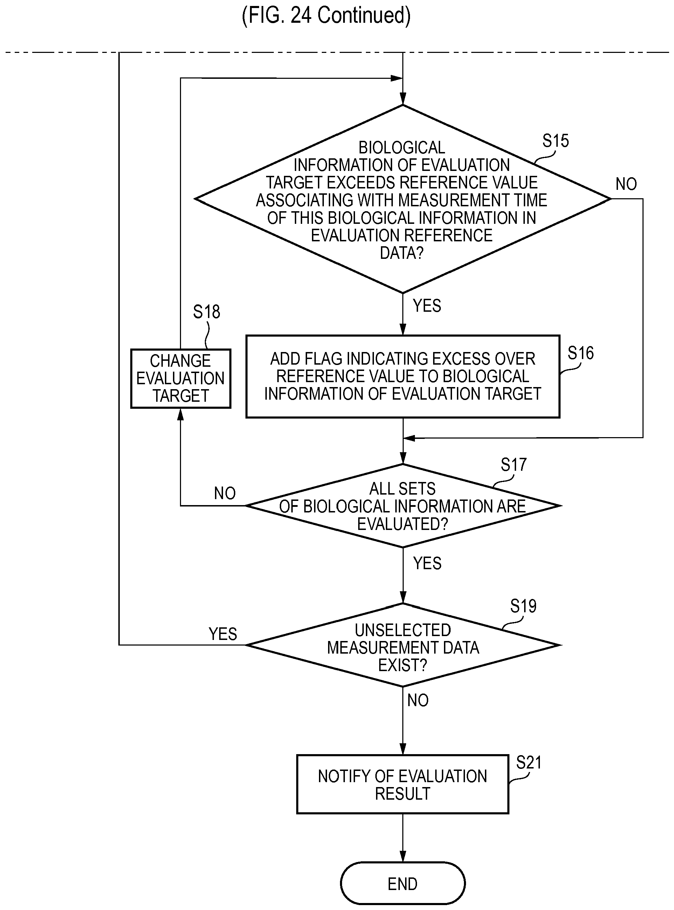

Fig. 24 is a flowchart illustrating the operation of the hospital terminal 4A of the biological information evaluation support system 100B shown in Fig. 22. The flowchart shown in Fig. 24 is identical with that shown in Fig. 14 except that step S11 is changed to step S11A, step S30 is added, step S12 is changed to step S12A, and step S13 is changed to step S13A.

Therefore, the processes identical with those of Fig. 14 are denoted by the same reference numerals, and their description is omitted. Here, it is premised that reference data for one day for at least respective days of week from Monday to Sunday are previously stored in the first storage section 13 of the biological information measurement device 1C.

First, the biological information measurement device 1C is brought into the hospital by the person to be measured, and connected to the hospital terminal 4A which is disposed in the hospital. In this state, the system controller 41 of the hospital terminal 4A reads the measurement data stored in the first storage section 13 of the biological information measurement device 1C, and causes the read measurement data to be stored in the third storage section 43 (step S11A).

Next, the reference data controller 410C of the hospital terminal 4A produces reference data associating with the respective day of week based on the measurement data for the respective day of week stored in the third storage section 43, and causes the reference data to be stored in the third storage section 43 (step S30).

Specifically, the reference data controller 410C of the hospital terminal 4A selects measurement data in which the month information is identical, and which includes the day of week information of Monday, from the measurement data stored in the third storage section 43. The reference data controller 410C of the hospital terminal 4A produces reference data associating with Monday based on the selected measurement data, and causes the month information contained in the selected measurement data and the produced reference data to be stored in the third storage section 43 while associating with each other.

The reference data controller 410C of the hospital terminal 4A performs similar processes while sequentially changing the day of week information in such a manner that the selected measurement data are measurement data in which the month information is identical, and which contain the day of week information of Tuesday, measurement data in which the month information is identical, and which contain the day of week information of Wednesday, ......, whereby reference data associating with the respective day of week information are produced.

Next, the reference data controller 410C of the hospital terminal 4A selects measurement data containing arbitrary month information and arbitrary day of week information, from the measurement data stored in the third storage section 43 (step S12A).

Next, the reference data controller 410C of the hospital terminal 4A reads reference data associating with the day of week information which is contained in the selected measurement data, and the month information associating with the reference data, from the third storage section 43.

Then, the reference data controller 410C of the hospital terminal 4A reads the correction value corresponding to the combination of the month information contained in the selected measurement data, and the month information associating with the read reference data, from the table of the third storage section 43, corrects the read reference data based on the read correction value, and sets the corrected reference data as evaluation reference data (step S13A).

After step S13A, the processes of step S14 and subsequent steps are performed. When the biological information measurement device 1C and the hospital terminal 4A are connected in the second or subsequent time, the process of step S30 may be omitted.

According to the biological information evaluation support system 100B, as described above, reference data can be produced by using only measurement data of an arbitrary person. Therefore, the construction of a system for collecting measurement data of a plurality of persons is not necessary, and the system cost can be reduced. Moreover, reference data can be produced in the hospital terminal 4A. Therefore, the function of the biological information measurement device 1C can be simplified, and the cost and power consumption of the biological information measurement device 1C can be reduced.

The function of the reference data controller 410C of the hospital terminal 4A may be changed in the following manner.

Namely, the reference data controller 410C produces one set of reference data based on measurement data for one day in which the month information and day of week information which are read from the biological information measurement device 1C are identical, and causes the produced reference data, and the month information and day of week information that are contained in the measurement data used in the production of the reference data, to be stored in the third storage section 43 while associating with each other.

When biological information is to be evaluated, then, the reference data controller 410C corrects the reference data stored in the third storage section 43, based on the month information and day of week information which are associated with the biological information that is the evaluation target, and sets the corrected reference data as the evaluation reference data.

In this case, a table storing correction values indicating degrees of increasing or decreasing reference values of reference data, with respect to combinations of the month information and day of week information which are associated with the reference data, and the month information and day of week information which are associated with the biological information that is the evaluation target is previously stored in the third storage section 43.

Based on the table, the reference data controller 410C then reads the correction value associating with the combination of the month information and day of week information associated with the biological information which is the evaluation target, and the month information and day of week information associated with the reference data, and corrects the reference data in accordance with the read correction value. Therefore, biological information can be evaluated in consideration of variation of biological information due to the difference in season and the difference in day of week.

According to the configuration, when biological information is measured only for one day, the biological information can be correctly evaluated. Therefore, this is effective in the case where biological information is to be evaluated in a hurry.

The biological information measurement device 1B shown in Fig. 18 is configured so as to produce reference data for each combination of the month information and the day of week information, and store it in the first storage section 13. In a modification of this, reference data are produced for each combination of the atmospheric temperature information and the day of week information, and stored in the first storage section 13. Hereinafter, the modification will be described.

In the modification, the biological information measurement device 1B has a temperature sensor which detects the ambient temperature (atmospheric temperature) of the device at a timing when biological information is measured. Moreover, the biological information measuring section 11A of the biological information measurement device 1B causes the measured biological information, the measurement time information of the biological information, date information and day of week information of the measurement time of the biological information, and the atmospheric temperature information (here, information of the atmospheric temperature itself) at the measurement time of the biological information, to be stored in the first storage section 13 while associating with one another.

When the measurement data which are measured by the biological information measuring section 11A, and which are stored in the first storage section 13 are accumulated for seven days, the reference data controller 110C of the biological information measurement device 1B of the modification produces reference data for each day of week based on the measurement data for seven days, and causes the produced reference data, and the mean value of the atmospheric temperature information at the measurement time of the biological information which is used in production of the reference data, to be stored in the first storage section 13 while associating with each other.

Specifically, the reference data controller 110C produces the typical value of the biological information belonging to each of the time period groups which are obtained by grouping the biological information contained in measurement data of one day that are measured on Mondays, by the "o'clock" information, as the reference value with respect to the time period group. As a result of the process, reference data associating with Monday are produced. The reference data controller 110C causes the reference data associating with Monday, and the mean value of the atmospheric temperature information contained in the measurement data used in production of the reference data, to be stored in the first storage section 13 while associating with each other.

The reference data controller 110C performs a similar process on measurement data which are measured on the other days of week, to produce and store reference data associating with each of Monday to Sunday.

When biological information is to be evaluated, the reference data controller 110C corrects reference data associating with the day of week information which is associated with the biological information that is the evaluation target, among reference data stored in the first storage section 13, based on the atmospheric temperature information which is associated with the biological information that is the evaluation target, and sets the corrected reference data as evaluation reference data.

When blood pressure information is exemplified as biological information, blood pressure information in a low atmospheric temperature tends to be high. Such atmospheric temperature variation is not considered in the reference data for each day of week which are produced by the reference data controller 110C, and which are stored in the first storage section 13.

Therefore, the reference data controller 110C corrects the evaluation reference data to data adequate for the atmospheric temperature at the measurement time, based on the relationship between the atmospheric temperature information at the measurement time of the biological information, and the mean value of the atmospheric temperature information associated with the evaluation reference data.

In order to realize such correction, a table which stores correction values indicating degrees of increasing or decreasing reference values of reference data, with respect to combinations of the mean value of atmospheric temperature information associated with reference data, and arbitrary atmospheric temperature information is previously stored in the first storage section 13.

Based on the table, the reference data controller 110C then reads the correction value associating with the combination of the atmospheric temperature information associated with the biological information which is the evaluation target, and the means value of the atmospheric temperature information associated with the reference data, and corrects the reference data in accordance with the read correction value. Therefore, biological information can be evaluated in consideration of variation of biological information due to the difference in atmospheric temperature.

Also in the biological information measurement device 1B of the modification, the function of the reference data controller 110C may be changed in the following manner.

When the measurement data which are measured by the biological information measuring section 11A, and which are stored in the first storage section 13 are accumulated for one day, namely, the reference data controller 110C produces one set of reference data based on measurement data for one day in which the day of week information is identical. Then, the reference data controller 110C causes the produced reference data, the mean value of the atmospheric temperature information at the measurement time of the biological information used in the production of the reference data, and the day of week information contained in measurement data used in the production of the reference data, to be stored in the first storage section 13 while associating with one another.

When biological information is to be evaluated, then, the reference data controller 110C corrects the reference data stored in the first storage section 13, based on the atmospheric temperature information and day of week information which are associated with the biological information that is the evaluation target, and sets the corrected reference data as the evaluation reference data.

In this case, a table storing correction values indicating degrees of increasing or decreasing reference values of reference data, with respect to combinations of the mean value of the atmospheric temperature information and day of week information which are associated with the reference data, and arbitrary atmospheric temperature information and day of week information is previously stored in the first storage section 13.

Based on the table, the reference data controller 110C reads a correction value corresponding to the combination of the atmospheric temperature information and day of week information which are associated with the biological information that is the evaluation target, and the mean value of the month information and day of week information which associate with the reference data, and corrects the reference data in accordance with the read correction value. This enables biological information to be evaluated in consideration of variation of biological information due to the difference in atmospheric temperature and the difference in day of week.

A configuration where, similarly in the descriptions with reference to Figs. 22 and 23, the hospital terminal has the functions of the reference-data controller 110C and biological information evaluating section 11D of the biological information measurement device 1B of the modification may be possible.

The above-described various programs are recorded on a non-transitory recording medium from which a computer can read the programs.

For example, such "computer readable recording medium" includes a ROM, an optical medium such as a CD-ROM (Compact Disc-ROM), a magnetic recording medium such as a memory card, and the like. Alternatively, such programs can be provided by downloading via a network.

The presently disclosed embodiments should be considered in all respects to be illustrative and not restrictive. The scope of the invention is indicated by the appended claims rather than the foregoing description, and all changes which come within the meaning and range of equivalents thereof are intended to be embraced therein.

As described above, the following matters are disclosed in the specification.

The disclosed biological information evaluation device is a biological information evaluation device which evaluates biological information measured from a person to be measured, and stored in a first storage section, the biological information evaluation device comprising: a biological information evaluating section which evaluates the biological information stored in the first storage section, based on reference data indicating a reference value of biological information in each of split time periods which are obtained by splitting one day into a plurality of time periods; and a reference data controller which controls the reference data to be used in evaluation of the biological information by the biological information evaluating section, based on at least calendar information at a measurement time of the biological information stored in the first storage section.

In the disclosed biological information evaluation device, the reference data controller controls the reference data based on the calendar information at the measurement time of the biological information stored in the first storage section, attribute information of the person to be measured, and atmospheric temperature information at the measurement time of the biological information.

In the disclosed biological information evaluation device, the reference data controller acquires reference data which associate with a combination of the calendar information and atmospheric temperature information at the measurement time of the biological information stored in the first storage section, and the attribute information of the person to be measured, from a second storage section which stores the reference data for each combination of calendar information, attribute information of a person, and atmospheric temperature information, and sets the acquired reference data as reference data to be used in evaluation of the biological information by the biological information evaluating section.

In the disclosed biological information evaluation device, the reference data stored in the second storage section are produced based on biological information of a plurality of persons, and, in the biological information of the plurality of persons, all attribute information of a person from whom the biological information is measured, calendar information at a measurement time of the biological information, and atmospheric temperature information at the measurement time of the biological information are identical.

In the disclosed biological information evaluation device, the reference data controller controls the reference data based on the calendar information at the measurement time of the biological information stored in the first storage section, and attribute information of the person to be measured.

In the disclosed biological information evaluation device, the reference data controller acquires reference data which associate with a combination of the calendar information at the measurement time of the biological information stored in the first storage section, and the attribute information of the person to be measured, from a second storage section which stores the reference data for each combination of calendar information and attribute information of a person, and sets the acquired reference data as reference data to be used in evaluation of the biological information by the biological information evaluating section.

In the disclosed biological information evaluation device, the reference data stored in the second storage section are produced based on biological information of a plurality of persons, and, in the biological information of the plurality of persons, all attribute information of a person from whom the biological information is measured, and calendar information at a measurement time of the biological information are identical.

In the disclosed biological information evaluation device, the reference data controller produces the reference data based on biological information for at least one day, measured by a biological information measuring section that measures biological information from the person to be measured, and stored in the first storage section, corrects the reference data based on month information and day of week information at the measurement time of the biological information stored in the first storage section, and sets the corrected reference data as reference data to be used in evaluation of the biological information by the biological information evaluating section.

In the disclosed biological information evaluation device, the reference data controller produces the reference data for each day of week based on biological information for seven days, measured by the biological information measuring section, and stored in the first storage section, corrects the reference data associating with the day of week information at the measurement time of the biological information stored in the first storage section, based on the month information at the measurement time of the biological information, and sets the corrected reference data as reference data to be used in evaluation of the biological information by the biological information evaluating section.

In the disclosed biological information evaluation device, the reference data controller produces the reference data based on biological information for at least one day, measured by a biological information measuring section that measures biological information from the person to be measured, and stored in the first storage section, corrects the reference data based on atmospheric temperature information and day of week information at the measurement time of the biological information stored in the first storage section, and sets the corrected reference data as reference data to be used in evaluation of the biological information by the biological information evaluating section.

In the disclosed biological information evaluation device, the reference data controller produces the reference data for each day of week based on biological information for seven days, measured by the biological information measuring section, and stored in the first storage section, corrects the reference data associating with the day of week information at the measurement time of the biological information stored in the first storage section, based on the atmospheric temperature information at the measurement time of the biological information, and sets the corrected reference data as reference data to be used in evaluation of the biological information by the biological information evaluating section.

The disclosed biological information measurement device comprises: the biological information evaluation device; and a biological information measuring section which measures biological information from the person to be measured, and which stores the biological information in the first storage section.

The disclosed biological information evaluation method is a biological information evaluation method for evaluating biological information measured from a person to be measured, and stored in a first storage section, the biological information evaluation method comprising: a biological information evaluating step of evaluating the biological information stored in the first storage section, based on reference data indicating a reference value of biological information in each of split time periods which are obtained by splitting one day into a plurality of time periods; and a reference data controlling step of controlling the reference data to be used in evaluation of the biological information in the biological information evaluating step, based on at least calendar information at a measurement time of the biological information stored in the first storage section.

The disclosed biological information evaluation program is a biological information evaluation program for evaluating biological information measured from a person to be measured, and stored in a first storage section, the biological information evaluation program which causes a computer to execute: a biological information evaluating step of evaluating the biological information stored in the first storage section, based on reference data indicating a reference value of biological information in each of split time periods which are obtained by splitting one day into a plurality of time periods; and a reference data controlling step of controlling the reference data to be used in evaluation of the biological information in the biological information evaluating step, based on at least calendar information at a measurement time of the biological information stored in the first storage section.

### Industrial Applicability

The invention is very convenient and effective particularly in application to a blood pressure monitor or the like.

Although the invention has been described with reference to the specific embodiments, the invention is not limited to the embodiments, and various changes can be made without departing from the technical spirit of the disclosed invention.

The application is based on Japanese Patent Application (No. 2016-020719) filed February 5, 2016, and its disclosure is incorporated herein by reference. Reference Signs List

- 100, 100A, 100B: biological information evaluation support system
- 1, 1A, 1B, 1C: biological information measurement device
- 2: biological information management device
- 3: network
- 4, 4A: hospital terminal
- 10: pulse wave detecting section
- 11, 21, 41: system controller
- 11A: biological information measuring section
- 11B: data transmission controller
- 11C, 41C, 110C, 410C: reference data controller
- 11D,41D: biological information evaluating section
- 12, 22, 42: communication interface
- 13: first storage section
- 14, 44: operating section
- 15,45: displaying section
- 21A: reference data producing section
- 21B: measurement data storage controller
- 23: second storage section
- 43: third storage section

## Claims

1. A biological information evaluation device which evaluates biological information measured from a person to be measured, and stored in a first storage section, the biological information evaluation device comprising:
a biological information evaluating section which evaluates the biological information stored in the first storage section, based on reference data indicating a reference value of biological information in each of split time periods which are obtained by splitting one day into a plurality of time periods; and
a reference data controller which controls the reference data to be used in evaluation of the biological information by the biological information evaluating section, based on at least calendar information at a measurement time of the biological information stored in the first storage section.

2. The biological information evaluation device according to claim 1, wherein
the reference data controller controls the reference data based on the calendar information at the measurement time of the biological information stored in the first storage section, attribute information of the person to be measured, and atmospheric temperature information at the measurement time of the biological information.

3. The biological information evaluation device according to claim 2, wherein
the reference data controller acquires reference data which associates with a combination of the calendar information and atmospheric temperature information at the measurement time of the biological information stored in the first storage section, and the attribute information of the person to be measured, from a second storage section which stores the reference data for each combination of calendar information, attribute information of a person, and atmospheric temperature information, and sets the acquired reference data as reference data to be used in evaluation of the biological information by the biological information evaluating section.

4. The biological information evaluation device according to claim 3, wherein
the reference data stored in the second storage section are produced based on biological information of a plurality of persons, and,
in the biological information of the plurality of persons, all attribute information of a person from whom the biological information is measured, calendar information at a measurement time of the biological information, and atmospheric temperature information at the measurement time of the biological information are identical.

5. The biological information evaluation device according to claim 1, wherein
the reference data controller controls the reference data based on the calendar information at the measurement time of the biological information stored in the first storage section, and attribute information of the person to be measured.

6. The biological information evaluation device according to claim 5, wherein
the reference data controller acquires reference data which associates with a combination of the calendar information at the measurement time of the biological information stored in the first storage section, and the attribute information of the person to be measured, from a second storage section which stores the reference data for each combination of calendar information and attribute information of a person, and sets the acquired reference data as reference data to be used in evaluation of the biological information by the biological information evaluating section.

7. The biological information evaluation device according to claim 6, wherein
the reference data stored in the second storage section are produced based on biological information of a plurality of persons, and,
in the biological information of the plurality of persons, all attribute information of a person from whom the biological information is measured, and calendar information at a measurement time of the biological information are identical.

8. The biological information evaluation device according to claim 1, wherein
the reference data controller produces the reference data based on biological information for at least one day, measured by a biological information measuring section that measures biological information from the person to be measured, and stored in the first storage section, corrects the reference data based on month information and day of week information at the measurement time of the biological information stored in the first storage section, and sets the corrected reference data as reference data to be used in evaluation of the biological information by the biological information evaluating section.

9. The biological information evaluation device according to claim 8, wherein
the reference data controller produces the reference data for each day of week based on biological information for seven days, measured by the biological information measuring section, and stored in the first storage section, corrects the reference data associating with the day of week information at the measurement time of the biological information stored in the first storage section, based on the month information at the measurement time of the biological information, and sets the corrected reference data as reference data to be used in evaluation of the biological information by the biological information evaluating section.

10. The biological information evaluation device according to claim 1, wherein
the reference data controller produces the reference data based on biological information for at least one day, measured by a biological information measuring section that measures biological information from the person to be measured, and stored in the first storage section, corrects the reference data based on atmospheric temperature information and day of week information at the measurement time of the biological information stored in the first storage section, and sets the corrected reference data as reference data to be used in evaluation of the biological information by the biological information evaluating section.

11. The biological information evaluation device according to claim 10, wherein
the reference data controller produces the reference data for each day of week based on biological information for seven days, measured by the biological information measuring section, and stored in the first storage section, corrects the reference data associating with the day of week information at the measurement time of the biological information stored in the first storage section, based on the atmospheric temperature information at the measurement time of the biological information, and sets the corrected reference data as reference data to be used in evaluation of the biological information by the biological information evaluating section.

12. A biological information measurement device comprising:
the biological information evaluation device according to any one of claims 1 to 11; and
a biological information measuring section which measures biological information from the person to be measured, and which stores the biological information in the first storage section.

13. A biological information evaluation method for evaluating biological information measured from a person to be measured, and stored in a first storage section, the biological information evaluation method comprising:
a biological information evaluating step of evaluating the biological information stored in the first storage section, based on reference data indicating a reference value of biological information in each of split time periods which are obtained by splitting one day into a plurality of time periods; and
a reference data controlling step of controlling the reference data to be used in evaluation of the biological information in the biological information evaluating step, based on at least calendar information at a measurement time of the biological information stored in the first storage section.

14. A biological information evaluation program for evaluating biological information measured from a person to be measured, and stored in a first storage section, the biological information evaluation program which causes a computer to execute:
a biological information evaluating step of evaluating the biological information stored in the first storage section, based on reference data indicating a reference value of biological information in each of split time periods which are obtained by splitting one day into a plurality of time periods; and
a reference data controlling step of controlling the reference data to be used in evaluation of the biological information in the biological information evaluating step, based on at least calendar information at a measurement time of the biological information stored in the first storage section.
